# EUROPÄISCHE PATENTSCHRIFT

(11) **EP 2 477 667 B1**
(45) Veröffentlichungstag und Bekanntmachung des Hinweises auf die Patenterteilung: **12.11.2014**
(21) Anmeldenummer: 10757729.8
(22) Anmeldetag: 13.09.2010
(51) Int. Cl.: A61L 27/42, A61L 27/46, A61L 27/52

(54) **GRANULATMISCHUNG, UMFASSEND ZWEI VERSCHIEDENE GRANULATE, ZUR KÜNSTLICHEN KALLUSDISTRAKTION**
GRANULATED MATERIAL MIXTURE COMPRISING TWO DIFFERENT GRANULATED MATERIALS FOR ARTIFICIAL CALLUS DISTRACTION
MÉLANGE DE GRANULÉS COMPRENANT DEUX TYPES DE GRANULÉS DIFFÉRENTS POUR DISTRACTION DE CAL ARTIFICIELLE

(30) Priorität: 14.09.2009 DE 102009042493
(43) Veröffentlichungstag der Anmeldung: 25.07.2012
(60) Teilanmeldung: 14181979.7
(73) Patentinhaber: CelGen3D AG, 6300 Zug (CH)
(72) Erfinder: HORVATH, Domonkos, 79798 Jestetten (DE)
(74) Vertreter: Wohlfahrt, Jan Günther
(86) Internationale Anmeldenummer: PCT/EP2010/005600
(87) Internationale Veröffentlichungsnummer: WO 2011/029622

(56) Entgegenhaltungen:
- WO-A1-2008/077257
- DE-A1- 19 706 667
- US-A1- 2002 169 506

## Beschreibung

Die vorliegende Erfindung betrifft eine Granulatmischung zur Regeneration eines Knochens, insbesondere mittels dreidimensionaler Distraktion, Verfahren zur dreidimensionalen Kallusdistraktion und Verwendungen der genannten Granulatmischung.

Knochenverluste werden heutzutage in der Regel mit Knochenersatzmaterialien oder mit auto- oder allogenem Knochen ausgefüllt.

Bei den Knochenersatzmaterialien handelt es sich beispielsweise um anorganische Materialien wie Kalziumphosphate, Hydroxylapatit oder Biogläser. Diese sollten nach langsamer Resorption mit Knochen ersetzt werden. Dies ist aber nur bei kleineren Defekten anwendbar, da sonst eine Infektionsgefahr wegen mangelnder Vaskularisierung besteht. Solche Knochenersatzmaterialien geben keine biomechanischen Impulse ab und lösen somit keine aktive Regeneration aus. Weiterhin gibt es synthetisch hergestellte Knochenersatzmaterialien aus organischen Materialien, wie Polyester, Polyaminosäuren, Polyanhydride, Polyorthoester, Polyphosphazene, Polylactide oder Polyglycolide oder aus allogenen organische Materialien, die beispielsweise bovinen Ursprungs sind. Knochensubstanzverluste können aber auch mit mikrovaskulären, angeschlossenen autogenen oder allogenen vaskularisierten Transplantaten überbrückt werden.

Aus biologischer Sicht ist ein autologes Spongiosatransplantat das beste Ersatzmaterial für einen Knochen. Solche Transplantate sind jedoch nur limitiert verfügbar und zeigen eine hohe Resorptionsrate nach der Transplantation.

Die im Stand der Technik eingesetzten Materialien und Techniken liefern häufig eine ungenügende Knochenqualität, so dass beispielsweise Implantatlager nicht fest verankert sind. Darüber hinaus ist der Knochenersatz häufig nicht genügend vaskularisiert, wodurch die Infektionsgefahr erhöht ist. Auch werden bei Verfahren aus dem Stand der Technik häufig Wachstumsfaktoren verwendet, die die Kosten für die Verfahren stark erhöhen.

Knochenersatzmaterialen werden häufig, insbesondere im Oral- und Kieferbereich, als Granulat eingesetzt. Ein solches Granulat ist beispielsweise in der WO 2006/010507 A2 beschrieben. Am Markt bekanntes Granulat ist beispielsweise Bio-Oss® der Firma Geistlich Pharma AG, BONIT matrix® der Firma DOT GmbH oder cyclOS® und Ceros® der Firma Mathys AG.

Die WO 2008/077257 A1 beschreibt eine Zusammensetzung, die feste, nicht verformbare Partikel sowie durch Flüssigkeitszusatz expandierbare Partikel enthält. Diese Zusammensetzung kann zur Regeneration von Knochen verwendet werden.

Die US 2002/169506 A1 beschreibt eine Zusammensetzung aus Granulat aus Calciumphosphat und aus partikulären, schwellbaren Polysacchariden, die zur Knochenregeneration verwendet werden kann.

Anstelle der Verwendung eines Knochenersatzes kann fehlende Knochensubstanz teilweise auch durch Knochenregeneration aufgefüllt werden. Segmentale Unterbrechungen der knöchernem Kontinuität an langen Röhrenknochen können so mittels Distraktionsosteogenese behandelt werden.

Die Kallusdistraktion ist bereits seit über hundert Jahren bekannt. Der wichtigste biologische Reiz für die Knochenbildung ist die mechanische Beanspruchung. Dadurch werden piezoelektrische Kräfte freigesetzt, die Osteoblasten und Osteoklasten aktivieren. Die Distraktionsosteogenese induziert die Knochenneubildung, indem durch langsame Trennung von Knochensegmenten biologische Wachstumsreize ausgelöst werden. Durch diese Methode wird die direkte Bildung von Geflechtsknochen durch Distraktion erreicht. Die definierte Zugspannung bei der Knochengeneration ist wesentlich. Legt man an Knochenfragmente eine solche definierte Zugspannung an, so zeigt das mesenchymale Gewebe im Spalt und an den angrenzenden Fragmentenden ein osteogenetisches Potential. Bei Vorliegen ausreichender vaskulärer Potenz kommt es unter progressiver Distraktion zur Metaplasie des organisierten Hämatoms, auch Blutkoagulat genannt, in einer Zone von longitudinal arrangiertem, fibrösem Gewebe, das sich unter optimalen externen und internen Bedingungen direkt in Geflechtsknochen umwandeln kann. Erschwerend ist jedoch, dass das Knochengewebe bei seiner Regeneration einer hoch-komplexen Steuerung unterliegt.

Die WO 01/91663 beschreibt eine zweidimensional orientierte Knochendistraktion mittels einer künstlichen Grenzfläche. Bei derartigen Distraktionsverfahren aus dem Stand der Technik ist häufig, beispielsweise im Kieferbereich, nur eine vertikale Regeneration möglich.

Somit ist eine Knochenregeneration durch Distraktion nicht bei jedem Knochendefekt anwendbar. Darüber hinaus sind die für eine Distraktion verwendeten Vorrichtungen komplex und die Distraktionsverfahren dauern vergleichsweise lang.

Die DE 10 2006 047 248 A1 beschreibt ein dreidimensionales Gerüst, das durch Volumenänderung Impulse direkt auf Osteoblasten überträgt und diese aktiviert.

Das der vorliegenden Erfindung zugrunde liegende technische Problem ist die Bereitstellung einer Vorrichtung, die es ermöglicht, Knochenregenerationsverfahren durchzuführen, die die Nachteile aus dem Stand der Technik überwinden. Das der Erfindung zugrunde liegende technische Problem ist auch die Bereitstellung von Vorrichtungen, die bereits bekannte Vorrichtungen zur Knochenregeneration verbessern, insbesondere in einfacher Weise verbessern.

Das der Erfindung zugrunde liegende technische Problem ist auch die Bereitstellung von Vorrichtungen, Verwendungen derselben und Verfahren, die eine einfache und kostengünstige Knochenregeneration erlauben.

Das der vorliegenden Erfindung zugrundeliegende technische Problem ist auch die Bereitstellung von Vorrichtungen, Verwendungen derselben und Verfahren, die es ermöglichen, Knochen zu regenerieren, die eine verbesserte Qualität aufweisen und genügend vaskularisiert sind.

Die vorliegende Erfindung löst das ihr zugrundeliegende technische Problem insbesondere durch die Bereitstellung von Vorrichtungen, Verfahren und Verwendungen gemäß der Patentansprüche.

Die erfindungsgemäßen Vorrichtungen sind insbesondere erfindungsgemäße Granulatgemische.

Die vorliegende Erfindung löst das ihr zugrunde liegende technische Problem insbesondere durch die Bereitstellung eines Granulatgemischs gemäß Anspruch 1, das zur Regeneration eines Knochens geeignet ist, und das mindestens ein dehnbares Partikel und mindestens ein nicht verformbares Partikel enthält.

Die vorliegende Erfindung löst das ihr zugrunde liegende technische Problem insbesondere auch durch die Bereitstellung eines erfindungsgemäßen Granulatgemischs, das zur Regeneration eines Knochens geeignet ist, und das mindestens ein dehnbares Partikel und mindestens ein nicht dehnbares Partikel enthält.

Erfindungsgemäß bevorzugt enthält das Granulatgemisch eine Vielzahl der dehnbaren Partikel und eine Vielzahl der nicht verformbaren Partikel.

Erfindungsgemäß bevorzugt enthält das Granulatgemisch eine Vielzahl der dehnbaren Partikel und eine Vielzahl der nicht dehnbaren Partikel.

Nicht erfindungsgemäß kann in einer alternativen Ausführungsform vorgesehen sein, dass das Granulatgemisch aus mindestens einem verformbaren Partikel und mindestens einem nicht verformbaren Partikel besteht.

Das erfindungsgemäße Granulatgemisch kann vorteilhafterweise in Verfahren, bevorzugt erfindungsgemäßen Verfahren, zur Knochen-regeneration, insbesondere zur dreidimensionalen Kallusdistraktion, verwendet werden.

Die vorliegende Lehre erfasst insbesondere Granulatgemische und Verfahren zur Knochenregeneration, wobei vorzugsweise Knochen im Kieferbereich und/oder im Parodontalbereich, regeneriert werden sollen.

Insbesondere versteht die vorliegende Erfindung unter dem Begriff der Knochenregeneration auch die Regeneration von Knochendefekten zum Beispiel nach Zystektomie, Tumorchirurgie oder Unfallchirurgie etc., unabhängig von der Topografie, und/oder insbesondere auch die Regeneration kleinerer Knochendefekte, die zum Beispiel durch Parodontitis entstehen.

Es können aber auch Knochen außerhalb des Kieferbereichs und/oder außerhalb des Parodontalbereichs regeneriert werden.

Erfindungsgemäß bevorzugt kann je nach Bedarf jegliches Mischungsverhältnis von den dehnbaren Partikeln und den nicht verformbaren Partikeln im Granulatgemisch vorgesehen sein.

Erfindungsgemäß bevorzugt kann je nach Bedarf jegliches Mischungsverhältnis von den dehnbaren Partikeln und den nicht dehnbaren Partikeln im Granulatgemisch vorgesehen sein.

Erfindungsgemäß bevorzugt beträgt das Mischungsverhältnis der dehnbaren Partikel und der nicht verformbaren Partikel in dem Granulatgemisch bezogen auf die Partikelanzahl von 1 zu 999 bis 999 zu 1. Erfindungsgemäß bevorzugt beträgt das Mischungsverhältnis der dehnbaren Partikel und der nicht verformbaren Partikel in dem Granulatgemisch bezogen auf die Partikelanzahl von 1 zu 99 bis 99 zu 1.

Es kann beispielsweise vorgesehen sein, dass das Mischungsverhältnis der dehnbaren Partikel und der nicht verformbaren Partikel in dem Granulatgemisch bezogen auf die Partikelanzahl von 1 zu 9 bis 9 zu 1 beträgt.

Erfindungsgemäß bevorzugt beträgt das Mischungsverhältnis der dehnbaren Partikel und der nicht dehnbaren Partikel in dem Granulatgemisch bezogen auf die Partikelanzahl von 1 zu 999 bis 999 zu 1. Erfindungsgemäß bevorzugt beträgt das Mischungsverhältnis der dehnbaren Partikel und der nicht dehnbaren Partikel in dem Granulatgemisch bezogen auf die Partikelanzahl von 1 zu 99 bis 99 zu 1.

Es kann beispielsweise vorgesehen sein, dass das Mischungsverhältnis der dehnbaren Partikel und der nicht dehnbaren Partikel in dem Granulatgemisch bezogen auf die Partikelanzahl von 1 zu 9 bis 9 zu 1 beträgt.

Es kann beispielsweise vorgesehen in einer alternativen Ausführungsform vorgesehen sein, dass mehr nicht verformbare Partikel als dehnbare, Partikel im Granulatgemisch vorliegen.

Erfindungsgemäß enthält das mindestens eine dehnbare Partikel ein Quellmittel. Es kann beispielsweise vorgesehen sein, dass das mindestens eine dehnbare Partikel aus einem Quellmittel besteht. Erfindungsgemäß enthält das mindestens eine dehnbare Partikel ein Quellmittel. Es kann beispielsweise vorgesehen sein, dass das mindestens eine dehnbare Partikel aus einem Quellmittel besteht.

In einer erfindungsgemäßen Alternative kann das Quellmittel fest sein. In einer erfindungsgemäßen Alternative kann das Quellmittel halbfest sein. In einer erfindungsgemäßen Alternative kann das Quellmittel als Schaum vorliegen. In einer erfindungsgemäßen Alternative kann das Quellmittel als Pulver vorliegen, insbesondere wenn das Quellmittel von einer Umhüllung oder Ummantelung umgeben ist. In einer erfindungsgemäßen Alternative kann das Quellmittel flüssig vorliegen, insbesondere wenn das Quellmittel von einer Ummantelung umgeben ist.

In einer erfindungsgemäßen Alternative kann das dehnbare Partikel fest sein. In einer erfindungsgemäßen Alternative kann das dehnbare Partikel halbfest sein. In einer erfindungsgemäßen Alternative kann das dehnbare Partikel als Schaum vorliegen. Das dehnbare Partikel kann insbesondere fest, halbfest oder schaumförmig vorliegen, wenn das dehnbare Partikel aus einem Quellmittel besteht.

Erfindungsgemäß bevorzugt ist das Quellmittel biokompatibel. Erfindungsgemäß bevorzugt ist das Quellmittel bioabbaubar.

Es kann beispielsweise vorgesehen sein, dass das Quellmittel nicht biogen ist, insbesondere dass das Quellmittel kein Kollagen enthält beziehungsweise kollagenfrei ist. Es kann aber auch vorgesehen sein, dass das Quellmittel biogen ist.

Es kann einerseits vorgesehen sein, dass das Quellmittel des mindestens einen dehnbaren Partikels von einer bioabbaubaren Umhüllung eingeschlossen ist. Die Umhüllung kann aus einem oder mehreren bioabbaubaren Materialien gebildet sein. Die nicht abgebaute, also intakte Umhüllung verhindert einen Kontakt des Quellmittels mit einer Flüssigkeit. Nachdem die Umhüllung teilweise oder ganz abgebaut ist, kann das Quellmittel dann in Kontakt mit einer Flüssigkeit kommen.

Dabei kann der Fachmann durch die Wahl der Dicke der Umhüllung die Zeit bestimmen, in der die Hülle aufgelöst wird. Dadurch ist die Vorbestimmung des Zeitpunkts des Beginns der Distraktion möglich. Je dicker beispielsweise die bioabbaubare Umhüllung ausgestaltet wird, umso später beginnt die Dehnung des umhüllten Granulats und damit der Beginn der Distraktionsimpulse.

Die Dicke der Umhüllung kann von der Dicke eines Molekularfilms bis zu 5 mm betragen. Erfindungsgemäß bevorzugt hat die Umhüllung mindestens eine Dicke eines Molekularfilms. Erfindungsgemäß bevorzugt hat die Umhüllung höchstens eine Dicke von 5 mm, insbesondere von 2mm, ganz besonders von 1 mm. In einer erfindungsgemäßen Ausführungsform hat die Umhüllung eine Dicke zwischen 0,1 µm und 1 mm. In einer erfindungsgemäßen Ausführungsform hat die Umhüllung eine Dicke von mindestens 10 µm und von höchstens 100 µm.

In einer erfindungsgemäßen Ausführungsform kann die Umhüllung zum Beispiel eine Resorptionszeit von mindestens einem Tag haben. In einer erfindungsgemäßen Ausführungsform kann die Umhüllung zum Beispiel eine Resorptionszeit von mindestens fünf Tagen haben. In einer erfindungsgemäßen Ausführungsform kann die Umhüllung zum Beispiel eine Resorptionszeit von etwa einer Woche haben. In einer erfindungsgemäßen Ausführungsform kann die Umhüllung zum Beispiel eine Resorptionszeit von 4 Tagen bis 10 Tagen haben. In einer erfindungsgemäßen Ausführungsform kann die Umhüllung zum Beispiel eine Resorptionszeit von 6 Tagen bis 8 Tagen haben. In einer erfindungsgemäßen Ausführungsform kann die Umhüllung zum Beispiel eine Resorptionszeit von höchstens 10 Wochen, insbesondere von höchstens 3 Wochen, haben. In einer erfindungsgemäßen Ausführungsform kann die Umhüllung zum Beispiel eine Resorptionszeit von mindestens einem Tag und höchstens zehn Wochen haben. In einer erfindungsgemäßen Ausführungsform kann die Umhüllung zum Beispiel eine Resorptionszeit von mindestens einem Tag und höchstens einer Woche haben. In einer erfindungsgemäßen Ausführungsform kann die Umhüllung zum Beispiel eine Resorptionszeit von mindestens einer Woche und höchstens zehn Wochen haben.

In einer erfindungsgemäßen Ausführungsform kann die Umhüllung zum Beispiel aus Gelatine bestehen oder Gelatine enthalten. In einer erfindungsgemäßen Ausführungsform kann die Umhüllung zum Beispiel aus Stoffen bestehen oder diese enthalten, die Gelatine-ähnlich sind oder die Eigenschaften von Gelatine haben. Dem Fachmann sind insbesondere aus der Galenik solche Stoffe bekannt.

Die Verwendung von Gelatine oder Gelatine-ähnlichen Stoffen hat den Vorteil, dass durch den Abbau der Gelatine der pH Wert der Umgebung nicht gesenkt wird, da keine sauren Abbauprodukte entstehen.

In einer erfindungsgemäßen Ausführungsform kann die Umhüllung zum Beispiel aus mindestens einem Gelatinefilm bestehen.

Es kann alternativ aber auch vorgesehen sein, dass das Quellmittel des mindestens einen dehnbaren Partikels von einer bioabbaubaren Ummantelung eingeschlossen ist. Erfindungsgemäß bevorzugt befindet sich das Quellmittel innerhalb der Ummantelung, wird also von der Ummantelung umschlossen. Erfindungsgemäß bevorzugt bildet also die Ummantelung einen Hohlraum, in dem sich das Quellmittel befindet. Erfindungsgemäß bevorzugt ist ein Teil des Hohlraums, insbesondere der ganze Hohlraum, der durch die Ummantelung gebildet wird, mit dem Quellmittel ausgefüllt. Erfindungsgemäß bevorzugt ist der ganze Hohlraum, der durch die Ummantelung gebildet wird, mit dem Quellmittel ausgefüllt. Der Hohlraum wird durch die Ummantelung begrenzt, auch wenn die Ummantelung Öffnungen, beispielsweise Poren aufweist.

Erfindungsgemäß bevorzugt reagiert die Ummantelung auf die Volumenänderung des Quellmittels durch Dehnung, Verformung und/oder Schrumpfung. Erfindungsgemäß bevorzugt reagiert die Ummantelung auf die Volumenänderung des Quellmittels durch Dehnung. Erfindungsgemäß bevorzugt reagiert die Ummantelung auf die Volumenänderung des Quellmittels durch Verformung. Erfindungsgemäß bevorzugt reagiert die Ummantelung auf die Volumenänderung des Quellmittels durch Dehnung und Verformung.

Erfindungsgemäß bevorzugt enthält die Ummantelung ein Material, ausgewählt aus der Gruppe bestehend aus Polyglykolsäure, Polymilchsäure, Poly(ε-caprolacton), Poly(β-hydroxybutyrat), Poly(p-dioxanon), einem Polyanhydrid oder einer Mischung von diesen, zum Beispiel einer Mischung von Polymilchsäure und Polyglykolsäure. Erfindungsgemäß bevorzugt enthält die Ummantelung Polymilchsäure. Erfindungsgemäß bevorzugt enthält die Ummantelung Poly(ε-caprolacton). Erfindungsgemäß bevorzugt enthält die Ummantelung ein Carbolacton.

Erfindungsgemäß bevorzugt enthält das Material der Ummantelung Copolymere, insbesondere aus zumindest zwei der vorgenannten Materialien. Erfindungsgemäß bevorzugt enthält das Material der Ummantelung Polymermischungen.

Erfindungsgemäß bevorzugt besteht die Ummantelung aus einem Material, ausgewählt aus der Gruppe bestehend aus Polyglykolsäure, Polymilchsäure, Poly(ε-caprolacton), Poly(β-hydroxybutyrat), Poly(p-dioxanon), einem Polyanhydrid oder einer Mischung von diesen. Erfindungsgemäß bevorzugt besteht das Material der Ummantelung aus Copolymeren aus zumindest zwei der vorgenannten Materialien.

Erfindungsgemäß bevorzugt besteht die Ummantelung aus Polymilchsäure. Eine Ummantelung, die Polymilchsäure enthält oder aus Polymilchsäure besteht hat den Vorteil, dass die Polymilchsäure zu kurzkettigen Metaboliten abgebaut wird. Darüber hinaus verleit Polymilchsäure der Ummantelung eine gewisse Härte.

Erfindungsgemäß bevorzugt besteht die Ummantelung aus Poly(ε-caprolacton). Eine Ummantelung, die Poly(ε-caprolacton) enthält oder aus Poly(ε-caprolacton) besteht hat den Vorteil, dass Poly(ε-caprolacton) besonders biokompatibel ist. Auch können lange Ketten aus Poly(ε-caprolacton) gebildet werden. Aus Poly(ε-caprolacton) werden bei der Zersetzung wenige bis keine freien Säuren gebildet.

Erfindungsgemäß bevorzugt besteht die Ummantelung aus Carbolacton.

Erfindungsgemäß bevorzugt besteht die Ummantelung aus mindestens einem Polymer oder enthält dieses, vorzugsweise aus räumlich vernetzten Polymeren.

Erfindungsgemäß bevorzugt besteht das Material der Ummantelung aus mindestens einem Faserverbundwerkstoff oder enthält dieses. Erfindungsgemäß bevorzugt besteht das Material der Ummantelung aus Fasern eines Faserverbundwerkstoffs oder enthält diese.

In einer erfindungsgemäßen Ausführungsform besteht die Ummantelung aus Gelatine oder Gelatine-ähnlichen Stoffen, oder enthält diese.

Erfindungsgemäß bevorzugt hat die Ummantelung mindestens eine zelladhäsive Eigenschaft, das heißt sie ist in der Lage, Zellen, insbesondere Osteoblasten, Fibroblasten und/oder Endothelzellen, zu binden, vorzugsweise spezifisch und selektiv zu binden. Erfindungsgemäß bevorzugt wird die zelladhäsive Eigenschaft der Ummantelung durch ihre Oberflächenbeschaffenheit bestimmt.

Erfindungsgemäß bevorzugt ist die Ummantelung vor dem Einbringen in einen Defektbereich eines Knochens außen mit Zellen, insbesondere Endothelzellen und/oder Osteoblasten und/oder Fibroblasten, beschichtet.

Erfindungsgemäß bevorzugt ist das Material der Ummantelung glatt. Erfindungsgemäß bevorzugt ist die Beschichtung der Ummantelung glatt. Erfindungsgemäß bevorzugt ist das Material der Ummantelung rau. Erfindungsgemäß bevorzugt ist die Beschichtung der Ummantelung rau. Durch eine erfindungsgemäß bevorzugte raue Oberfläche steht eine größere Oberfläche zur Bindung der Osteoblasten zur Verfügung.

Erfindungsgemäß bevorzugt ist die Ummantelung mit Hydroxylapatit beschichtet. Eine erfindungsgemäß bevorzugte Beschichtung mit Hydroxylapatit ermöglicht eine bindungsfördernde Adsorption von Proteinen.

Erfindungsgemäß bevorzugt ist die Ummantelung mit einem Hydrogel beschichtet. Erfindungsgemäß bevorzugt ist die Hydrogelschicht dünn.

Erfindungsgemäß bevorzugt ist die Ummantelung mit mindestens einem Protein beschichtet. Erfindungsgemäß bevorzugt enthält das mindestens eine Protein die Aminosäuresequenz Arg-Gly-Asp, also RGD. Erfindungsgemäß bevorzugt ist die Ummantelung mit mindestens einem Peptid beschichtet. Erfindungsgemäß bevorzugt ist das mindestens eine Peptid ein Peptid, das die Zelladhäsion initiiert. Erfindungsgemäß bevorzugt ist das mindesten eine Peptid ein RGD-Peptid. Erfindungsgemäß bevorzugt ist das mindestens eine Peptid synthetisch hergestellt. Erfindungsgemäß bevorzugt enthält das mindestens eine Peptid die Aminosäuresequenz Arg-Gly-Asp, also RGD. Erfindungsgemäß bevorzugt besteht das mindestens eine Peptid aus der Aminosäuresequenz Arg-Gly-Asp, also RGD.

Erfindungsgemäß bevorzugt ist die Ummantelung mit sternförmigen Polyethylen-Glykol-Polymeren (Star-PEG) beschichtet.

Erfindungsgemäß bevorzugt ist das mindestens eine Protein an die Polyethylen-Glykol-Polymerbeschichtung, besonders bevorzugt kovalent, gebunden. Erfindungsgemäß bevorzugt ist das mindestens eine Peptid an die Polyethylen-Glykol-Polymerbeschichtung, besonders bevorzugt kovalent, gebunden.

Die Adhäsion von Osteoblasten ist ein Rezeptor-vermittelter Kontakt zwischen den Molekülen der extrazellulären Matrix und den Aktinfasern des Cytoskeletts. Diese Region wird auch als fokale Kontaktzone bezeichnet. In den fokalen Kontakten sind sowohl Moleküle, die für eine Bindung sorgen, als auch Moleküle, die für eine Signaltransduktion verantwortlich sind. Die Ausbildung der fokalen Adhäsion ist hauptsächlich durch Integrine bedingt. Die Integrine unterscheiden sich durch ihre Bioaffinität von anderen Zelloberflächenrezeptoren. Adhäsionsproteine in Form von ultradünner Beschichtung auf der Ummantelung erleichtern die Adhäsionsbindung von Osteoblasten an die erfindungsgemäße Vorrichtung. Fibronektin ist ein extrazelluläres Adhäsionsprotein mit mehreren spezifischen Bindungsstellen für Rezeptoren und dient somit zur Bindung der Osteoblasten an die extrazelluläre Matrix. Fibronektin ist ein großes Glykoprotein, das als Dimer aus zwei nahezu identischen Untereinheiten besteht. Fibronektin besteht aus etwa 90 Aminosäuren. Die zellbindende Stelle von Fibronektin wurde als Tripeptidsequenz Arg-Gly-Asp (RGD) identifiziert.

Erfindungsgemäß bevorzugt ist die Oberfläche der Ummantelung chemisch modifiziert. Erfindungsgemäß bevorzugt ist die Oberfläche der Ummantelung durch reaktive Moleküle oder Molekülgruppen chemisch modifiziert. Erfindungsgemäß bevorzugt können die Moleküle oder Molekülgruppen, mit denen die Oberfläche der Ummantelung chemisch modifiziert ist, mit Ankerproteinen der extrazellulären Matrix von Zellen reagieren. Erfindungsgemäß bevorzugt ist die Oberfläche der Ummantelung hydrophil. Hydrophile Oberflächen erlauben eine bessere Adhäsion für Zellen als hydrophobe.

Erfindungsgemäß bevorzugt hat die Ummantelung eine Stärke von mindestens 0,01 mm. Erfindungsgemäß bevorzugt hat die Ummantelung eine Stärke von höchstens 1 mm. Erfindungsgemäß bevorzugt hat die Ummantelung eine Stärke von mindestens 0,05 mm und höchstens 0,5 mm. Erfindungsgemäß bevorzugt hat die Ummantelung eine Stärke von etwa 0,1 mm.

Erfindungsgemäß bevorzugt ist die Ummantelung für eine Flüssigkeit durchlässig. Erfindungsgemäß bevorzugt ist die Ummantelung für Wasser durchlässig. Erfindungsgemäß bevorzugt ist die Ummantelung porös. Erfindungsgemäß bevorzugt hat die Ummantelung Poren, die für Wasser und für Feststoffe, beispielsweise Proteine und Zucker, mit einer Masse von unter 100 kDa, besonders bevorzugt von unter 50 kDa, durchlässig sind. Erfindungsgemäß bevorzugt hat die Ummantelung Poren, die für Feststoffe, beispielsweise Proteine und Zucker, mit einer Masse von über 50 kDa, besonders bevorzugt von über 100 kDa, insbesondere von über 150 kDa, nicht durchlässig sind. Erfindungsgemäß bevorzugt haben die Poren eine Größe von höchstens 2 µm, besonders bevorzugt von höchstens 1 µm. Erfindungsgemäß bevorzugt haben die Poren eine Größe von höchstens 0,5 µm, besonders bevorzugt von höchstens 0,1 µm. Erfindungsgemäß bevorzugt haben die Poren eine Größe von mindestens 0,01 µm, besonders bevorzugt von mindestens 0,05 µm. Erfindungsgemäß bevorzugt haben die Poren eine Größe von mindestens 0,1 µm, besonders bevorzugt von mindestens 0, 5 µm. Erfindungsgemäß bevorzugt haben die Poren eine Größe von 1 µm.

Erfindungsgemäß bevorzugt hat mindestens ein Teil der Ummantelung die Form eines Faltenbalgs.

Erfindungsgemäß bevorzugt hat mindestens ein Teil der Ummantelung die Form eines Faltenschlauchs.

Erfindungsgemäß bevorzugt hat die Ummantelung die Form eines Faltenbalgs.

Erfindungsgemäß bevorzugt hat die Ummantelung die Form eines Faltenschlauchs.

Der als Faltenbalg oder Faltenschlauch geformte Teil der Ummantelung kann wie der gleichartige Teil eines Knickstrohhalms auseinandergezogen oder zusammengeschoben werden.

Der Faltenbalg oder Faltenschlauch besteht bevorzugt aus mindestens einer, besonders bevorzugt mindestens zwei, insbesondere einer Vielzahl von Falten.

Erfindungsgemäß bevorzugt haben die Falten des Faltenbalgs oder Faltenschlauchs eine Länge von 0,5 mm bis 2 mm, gerechnet vom inneren Umfang der Ummantelung hin zum distalen Ende der Falten, die gleichsam den äußeren Umfang bilden. Erfindungsgemäß bevorzugt haben die Falten des Faltenbalgs oder Faltenschlauchs eine Länge von 1 mm.

Erfindungsgemäß bevorzugt ist der mindestens eine als Faltenbalg oder Faltenschlauch geformte Teil der Ummantelung im Ausgangszustand, also vor Verwendung der Partikel, zusammengeschoben. Durch die Volumenänderung, insbesondere Volumenvergrößerung des Quellmittels wird der bevorzugte mindestens eine als Faltenbalg oder -schlauch geformte Teil der Ummantelung auseinandergeschoben.

Erfindungsgemäß bevorzugt ist die Außenfläche der Ummantelung, insbesondere durch Anordnung von Konturen, vergrößert. Diese Vergrößerung erhöht nicht nur die für die Zellen verfügbare Oberfläche, sondern beeinflusst auch die Organisation des Zellwachstums.

Erfindungsgemäß bevorzugt ist die Außenfläche der Ummantelung durch Lamellen vergrößert. In bevorzugter Ausführungsform der vorliegenden Erfindung sind die Lamellen stäbchen- oder röhrenartig ausgebildete Fortsätze. In einer weiteren, besonders bevorzugten Ausführungsform sind die Lamellen flächig ausgebildete Fortsätze, insbesondere wandartige, plättchenartige, blattartige, fächerartige, flügelartige oder sonstig flächig ausgebildete Fortsätze. In einer weiteren bevorzugten Ausführungsform sind die Lamellen oberflächenvergrößert, insbesondere durch Lamellenunterstrukturen, Verzweigungen, Ausstülpungen oder netzartigen Strukturen:

Erfindungsgemäß bevorzugt trägt die Außenseite der Ummantelung mindestens eine Lamelle. Erfindungsgemäß bevorzugt trägt die Au-ßenseite der Ummantelung mindestens zwei Lamellen. Erfindungsgemäß bevorzugt trägt die Außenseite der Ummantelung eine Vielzahl von Lamellen. Erfindungsgemäß bevorzugt trägt die Außenseite der Ummantelung 2 bis 20 Lamellen.

Erfindungsgemäß bevorzugt kann die mindestens eine Lamelle Bestandteil der Ummantelung sein. Erfindungsgemäß bevorzugt besteht die mindestens eine Lamelle aus dem gleichen Material wie die Ummantelung.

Erfindungsgemäß bevorzugt ist die mindestens eine Lamelle nicht Bestandteil der Ummantelung. Erfindungsgemäß bevorzugt besteht die mindestens eine Lamelle aus einem anderen Material als die Ummantelung.

Erfindungsgemäß bevorzugt ist die Ummantelung biokompatibel. Erfindungsgemäß ist die Ummantelung bioabbaubar. Erfindungsgemäß bevorzugt sind die Ummantelung und/oder das Quellmittel bioabbaubar.

Im Zusammenhang mit der vorliegenden Erfindung bedeutet "bioabbaubar", dass das Material durch Hydrolyse, Polymerauflösung, enzymatische Degradation, und/oder Dissoziation der Materialbestandteile degradiert beziehungsweise resorbiert werden kann, vorzugsweise in einen Organsimus, zum Beispiel einem menschlichen oder tierischen Organismus. Erfindungsgemäß bevorzugt haben die Degradationsprodukte der Partikel ein Molekulargewicht von höchstens 50000 g/mol, besonders bevorzugt von höchstens 40000 g/mol. Somit können sie auf normalem Weg ausgeschieden werden.

Erfindungsgemäß bevorzugt werden die bioabbaubare, verformbare Partikel in einem Organismus innerhalb einer Resorptionszeit von zwei Jahren, besonders bevorzugt innerhalb von einem Jahr, insbesondere innerhalb von einem Monat, am meisten bevorzugt innerhalb von zwei Wochen, abgebaut.

Erfindungsgemäß bevorzugt beginnt die Resorption nach 6 Wochen nach dem Einbringen der Partikel in einen Organismus.

Erfindungsgemäß besonders bevorzugt beträgt die Resorptionszeit der bioabbaubaren, dehnbaren Partikel, insbesondere der Ummantelung und/oder des Quellmittels, mindestens vier Wochen, besonders bevorzugt mindestens acht Wochen, insbesondere sechzehn Wochen, am meisten bevorzugt mindestens zweiunddreißig Wochen. Erfindungsgemäß bevorzugt beträgt die Resorptionszeit der bioabbaubaren, dehnbaren Partikel höchstens zweiundfünfzig Wochen, besonders bevorzugt höchstens achtunddreißig Wochen, stärker bevorzugt höchstens sechzehn Wochen, am meisten bevorzugt höchstens acht Wochen.

Erfindungsgemäß bevorzugt sind die bioabbaubaren, dehnbaren Partikel biodegradierbar. Erfindungsgemäß bevorzugt sind die Komponenten der Vorrichtung, insbesondere die Ummantelung und das Quellmittel, biodegradierbar.

In einer erfindungsgemäßen alternativen Ausführungsform sind die dehnbaren Partikel porös. In einer erfindungsgemäßen alternativen Ausführungsform sind die dehnbaren Partikel nicht porös.

Es kann in einer Ausführungsform vorgesehen sein, dass das Quellmittel ein Hydrogel ist.

Erfindungsgemäß bevorzugt ist das Hydrogel Carboxymethylzellulose. Erfindungsgemäß bevorzugt enthält das Hydrogel Carboxymethylzellulose. Erfindungsgemäß bevorzugt besteht das Hydrogel aus einem Polysaccharid. Erfindungsgemäß bevorzugt enthält das Hydrogel mindestens ein Polysaccharid. Erfindungsgemäß bevorzugt ist das Hydrogel Hyaluronsäure. Erfindungsgemäß bevorzugt enthält das Hydrogel Hyaluronsäure. Erfindungsgemäß bevorzugt enthält das Quellmittel verschiedene Komponenten, insbesondere Mischungen aus den hier offenbarten Komponenten wie Carboxymethylzellulose, Polysacchariden und/oder Hyaluronsäure.

In einer erfindungsgemäßen Ausführungsform kann das Hydrogel Polyethylenglykol (PEG) sein. In einer erfindungsgemäßen Ausführungsform kann das Hydrogel Polyethylenglykol (PEG) enthalten. In einer erfindungsgemäßen Ausführungsform kann das Hydrogel Polyacrylamid sein. In einer erfindungsgemäßen Ausführungsform kann das Hydrogel Polyacrylamid enthalten.

Es kann in einer Ausführungsform insbesondere vorgesehen sein, dass das Quellmittel des mindestens einen dehnbaren Partikels aus einem Polysaccharid besteht.

Es kann in einer Ausführungsform insbesondere vorgesehen sein, dass das Quellmittel des mindestens einen dehnbaren Partikels aus einem Glucosamin besteht.

Es kann in einer Ausführungsform insbesondere vorgesehen sein, dass die Vielzahl der dehnbaren Partikel aus dem gleichen oder aus unterschiedlichen Materialien bestehen.

Erfindungsgemäß bevorzugt wird die Verformung, insbesondere Dehnung des Quellmittels durch die Aufnahme von Flüssigkeit, bevorzugt von einer Flüssigkeit enthaltend Biomoleküle und/oder Zellen, besonders bevorzugt Blut, durch das Quellmittel ausgelöst.

Erfindungsgemäß bevorzugt hat das mindestens eine dehnbare Partikel eine Partikelgröße von 0,1 µm bis 10 mm.

In einer erfindungsgemäßen alternativen Ausführungsform hat das mindestens eine dehnbare Partikel eine Partikelgröße von 0,1 mm bis 10 mm.

In einer erfindungsgemäßen alternativen Ausführungsform hat das mindestens eine dehnbaren Partikel eine Partikelgröße von mindestens 0,1 µm. In einer erfindungsgemäßen alternativen Ausführungsform hat das mindestens ein dehnbare Partikel eine Partikelgröße von mindestens 1 µm. In einer erfindungsgemäßen alternativen Ausführungsform hat das mindestens eine dehnbare Partikel eine Partikelgröße von mindestens 10 µm. In einer erfindungsgemäßen alternativen Ausführungsform hat das mindestens eine dehnbare Partikel eine Partikelgröße von mindestens 100 µm. In einer erfindungsgemäßen alternativen Ausführungsform hat das mindestens eine dehnbare Partikel eine Partikelgröße von mindestens 200 µm.

In einer erfindungsgemäßen alternativen Ausführungsform hat das mindestens eine dehnbare Partikel eine Partikelgröße von höchstens 10 mm. In einer erfindungsgemäßen alternativen Ausführungsform hat das mindestens eine dehnbare Partikel eine Partikelgröße von höchstens 5 mm. In einer erfindungsgemäßen alternativen Ausführungsform hat das mindestens eine dehnbare Partikel eine Partikelgröße von höchstens 1 mm.

In einer bevorzugten Ausführungsform beträgt die Partikelgröße der dehnbaren und der nicht verformbaren Partikel mindestens 0,0001 mm. In einer bevorzugten Ausführungsform beträgt die Partikelgröße der dehnbaren und der nicht verformbaren Partikel mindestens 0,001 mm. In einer bevorzugten Ausführungsform beträgt die Partikelgröße der dehnbaren und der nicht verformbaren Partikel mindestens 0,01 mm.

In einer bevorzugten Ausführungsform sind die dehnbaren und/oder die nicht verformbaren Partikel nicht pulverförmig sondern granulatförmig. In einer bevorzugten Ausführungsform sind die dehnbaren und die nicht verformbaren Partikel nicht pulverförmig sondern granulatförmig.

In einer erfindungsgemäßen Ausführungsform beträgt die Partikelgröße 0,2 mm bis 5 mm. In einer erfindungsgemäßen Ausführungsform beträgt die Partikelgröße 0,5 mm bis 5 mm. In einer erfindungsgemäßen Ausführungsform beträgt die Partikelgröße 0,6 mm bis 5 mm. In einer erfindungsgemäßen Ausführungsform beträgt die Partikelgröße 0,5 mm bis 4 mm.

In einer bevorzugten Ausführungsform beträgt die Partikelgröße mindestens 0,001 mm. In einer bevorzugten Ausführungsform beträgt die Partikelgröße mindestens 0,01 mm.

In einer erfindungsgemäßen Ausführungsform beträgt die Partikelgröße mindestens 0,0001 mm. In einer erfindungsgemäßen Ausführungsform beträgt die Partikelgröße mindestens 0,1 mm. In einer erfindungsgemäßen Ausführungsform beträgt die Partikelgröße mindestens 0,2 mm. In einer erfindungsgemäßen Ausführungsform beträgt die Partikelgröße mindestens 0,3 mm. In einer erfindungsgemäßen Ausführungsform beträgt die Partikelgröße mindestens 0,4 mm. In einer erfindungsgemäßen Ausführungsform beträgt die Partikelgröße mindestens 0,5 mm. In einer erfindungsgemäßen Ausführungsform beträgt die Partikelgröße mindestens 0,6 mm.

In einer erfindungsgemäßen Ausführungsform beträgt die Partikelgröße höchstens 10 mm. In einer erfindungsgemäßen Ausführungsform beträgt die Partikelgröße höchstens 5 mm. In einer erfindungsgemäßen Ausführungsform beträgt die Partikelgröße höchstens 4 mm. In einer erfindungsgemäßen Ausführungsform beträgt die Partikelgröße höchstens 2 mm. In einer erfindungsgemäßen Ausführungsform beträgt die Partikelgröße höchstens 1 mm.

Die Angaben der Partikelgrößen der dehnbaren Partikel beziehen sich auf die Größe der Partikel im Ausgangszustand, also im ungedehnten Zustand.

Es kann in einer alternativen Ausführungsform insbesondere vorgesehen sein, dass die Partikel der Vielzahl der dehnbaren Partikel in einer einzigen Partikelgröße vorliegen.

Es kann in einer alternativen Ausführungsform aber auch vorgesehen sein, dass die Partikel der Vielzahl der dehnbaren Partikel mindestens in zwei verschiedenen Partikelgrößen vorliegen. Es kann in einer alternativen Ausführungsform insbesondere auch vorgesehen sein, dass die Partikel der Vielzahl der dehnbaren Partikel in zwei verschiedenen Partikelgrößen vorliegen. Es kann in einer alternativen Ausführungsform insbesondere auch vorgesehen sein, dass die Partikel der Vielzahl der dehnbaren Partikel in drei verschiedenen Partikelgrößen vorliegen. Es kann in einer alternativen Ausführungsform insbesondere auch vorgesehen sein, dass die Partikel der Vielzahl der dehnbaren Partikel in vier verschiedenen Partikelgrößen vorliegen. Es kann in einer alternativen Ausführungsform insbesondere auch vorgesehen sein, dass die Partikel der Vielzahl der dehnbaren Partikel in fünf verschiedenen Partikelgrößen vorliegen.

Es kann in einer alternativen Ausführungsform insbesondere auch vorgesehen sein, dass die Partikel der Vielzahl der dehnbaren Partikel der Granulatmischung in eins bis zehn, insbesondere in eins bis fünf, aber auch in zwei bis zehn, insbesondere in zwei bis fünf, verschiedenen Partikelgrößen vorliegen.

Es kann in einer alternativen Ausführungsform insbesondere auch vorgesehen sein, dass die Partikel der Vielzahl der dehnbaren Partikel der Granulatmischung in einer Vielzahl verschiedener Partikelgrößen vorliegen.

Erfindungsgemäß ist das mindestens eine verformbare Partikel dehnbar. Erfindungsgemäß ist das mindestens eine verformbare Partikel dehnbar. Erfindungsgemäß ist das mindestens eine verformbare Partikel dehnbar

Erfindungsgemäß ist das mindestens eine verformbare Partikel dehnbar.

Erfindungsgemäß handelt es sich bei den verformbaren Partikeln um dehnbare Partikel.

Erfindungsgemäß bevorzugt sind die verformbaren Partikel vordefiniert und kontrolliert in Abhängigkeit von der Krafteinwirkung dehnbar.

Erfindungsgemäß bevorzugt erfolgt die Verformung des mindestens eines verformbaren Partikels durch eine Volumenveränderung des Partikels.

Im Zusammenhang mit der vorliegenden Erfindung wird unter dem "Volumen" der verformbaren Partikel, und insbesondere des Quellmittels, das Volumen verstanden, das durch die die Außenflächen der Partikel bzw. des Quellmittels begrenzt wird. Die verformbaren Partikel liegen in bevorzugter Form in einem, vorzugsweise ursprünglichen, Ausgangsvolumen vor, das sich durch Kontakt mit einer Flüssigkeit, insbesondere durch Flüssigkeitsaufnahme, zu einem anderen Volumen ändern kann. Eine Änderung des Volumens bedeutet eine Änderung des Ausgangsvolumens, insbesondere eine signifikante Änderung des Ausgangsvolumens, bevorzugt eine Vergrößerung des Ausgangsvolumens. Die Änderung kann zum Beispiel eine Änderung des Ausgangsvolumens von mindestens 1%, bevorzugt von 5%, bevorzugt von 10%, bevorzugt von 15%, bevorzugt von 20%, bevorzugt von 30%, bevorzugt von 40%, bevorzugt von 50%, bevorzugt von 60%, bevorzugt von 70%, bevorzugt von 80%, bevorzugt von 90%, im Fall einer Vergrößerung bevorzugt von mindestens 100%, bevorzugt von 150%, bevorzugt von 200% oder bevorzugt von 300%, beispielsweise durch Dehnung der Partikel, sein.

In einer erfindungsgemäßen Ausführungsform findet die Dehnung eines verformbaren Partikels in alle drei Raumrichtung statt. In einer alternativen erfindungsgemäßen Ausführungsform findet die Dehnung gerichtet in eine oder zwei Raumrichtungen statt. In einer alternativen erfindungsgemäßen Ausführungsform findet die Dehnung gerichtet in eine Raumrichtung statt. Eine gerichtete Dehnung kann zum Beispiel durch eine Ummantelung, insbesondere eine Ummantelung mit einem Faltenbalg, erreicht werden. Die gerichtete Dehnung kann zum Beispiel aber auch durch eine Umhüllung erreicht werden, die an verschiedenen Stellen unterschiedlich dick ist und sich daher unterschiedlich schnell auflöst. Dann findet eine Dehnung an Bereichen des Quellmittels statt, an den die Umhüllung schon aufgelöst ist und nicht an Stellen, an denen die Umhüllung noch nicht aufgelöst ist.

Erfindungsgemäß bevorzugt wird die Volumenveränderung die verformbaren Partikel durch den Kontakt mit und die Aufnahme von Flüssigkeit, bevorzugt von einer Flüssigkeit enthaltend Biomoleküle und/oder Zellen, besonders bevorzugt Blut, durch die Partikel, beispielsweise durch das Quellmittel ausgelöst wird. Erfindungsgemäß bevorzugt ist die Flüssigkeit Wasser. Erfindungsgemäß bevorzugt ist die Flüssigkeit eine Körperflüssigkeit. Erfindungsgemäß bevorzugt ist die Flüssigkeit eine interstitielle Flüssigkeit. Erfindungsgemäß bevorzugt ist die Flüssigkeit Blut. Erfindungsgemäß bevorzugt enthält die aufgenommene Flüssigkeit keine festen Bestandteile über 150 kDa, besonders bevorzugt über 100 kDa, insbesondere über 50 kDa.

Erfindungsgemäß bevorzugt ist die Volumenänderung der verformbaren Partikel eine Volumenvergrößerung.

Durch die Bereitstellung des erfindungsgemäßen Granulatgemischs wird es ermöglicht, dieses in einen Knochendefekt einzubringen, beispielsweise chirurgisch einzubringen. Nach Einbringen in den Knochendefekt ist erfindungsgemäß vorgesehen, dass sich das Volumen die verformbaren Partikel aufgrund eines Kontakts mit einer Flüssigkeit und einer damit verbundenen Flüssigkeitswanderung insbesondere einer Aufnahme der Flüssigkeit ändert, zum Beispiel vergrößert oder verkleinert, insbesondere aber ausdehnt. Durch die Volumenänderung der verformbaren Partikel werden die verformbaren Partikel in ihrer Form und/oder Größe verändert, besonders bevorzugt die Oberfläche und damit das umschlossene Volumen vergrößert. Dies bewirkt, dass nach dem Einbringen des Granulatgemischs in den Knochendefekt eingewanderte und an die Partikel, insbesondere an die verformbaren Partikel und an die nicht verformbaren Partikel, adhärierte osteogene Zellen oder Zellverbünde langsam und definiert, insbesondere sofern sie zu den Partikeln einen distraktionswirksamen Abstand haben, einer Spannung, also einem biomechanischen Reiz, ausgesetzt werden. Durch die definierte Ausdehnung der verformbaren Partikel im Knochendefekt und der damit verbundenen Bewegung von den nicht verformbaren Partikeln und der damit verbundenen Distraktion von an den Partikeln adhärierten Zellen wird eine dreidimensionale Kallusdistraktion erzielt. Dadurch entsteht in dem gesamten Defekt durch Distraktion auf einmal die Vorstufe eines Kallus, der lediglich noch verknöchern muss. Vorteilhafterweise wird dieser Reiz im Wesentlichen viele Zellen, besonders bevorzugt alle Zellen gleichzeitig erreichen. Erfindungsgemäß ist es möglich, auf die Osteoblasten direkt biomechanische Reize zu übertragen, ohne dass Fibroblasten notwendig sind. Die Distraktion kann also auf die Osteoblasten mit vergleichsweise kleinen Kräften wirken. Ohne an die Theorie gebunden zu sein werden die Distraktionsimpulse an die meisten Osteoblasten über die nicht verformbaren Partikel übertragen, insbesondere wenn in dem Granulatgemisch mehr nicht verformbare Partikel als verformbare Partikel vorliegen und/oder wenn die nicht verformbaren Partikel größer sind als die nicht verformbaren Partikel.

Das erfindungsgemäße Granulatgemisch kann vorteilhafterweise in Verfahren, bevorzugt erfindungsgemäßen Verfahren, zur Knochenregeneration, insbesondere zur dreidimensionalen Kallusdistraktion, verwendet werden.

Erfindungsgemäß bevorzugt überträgt das erfindungsgemäße Granulatgemisch biomechanische Impulse, insbesondere Dehnungsreize oder Druckreize, auf die überträgt das Granulatgemisch umgebenden Zellen, so dass diese um Distanzen von mindestens 0,5 µm, insbesondere von 1 µm, mehr bevorzugt von 2 µm, am meisten bevorzugt von 10 µm bis bevorzugt 100 µm, insbesondere besonders bevorzugt 1000 µm, besonders bevorzugt 1 cm, am meisten bevorzugt bis zu 10 cm distrahiert oder zusammengedrückt werden. Das erfindungsgemäße Granulatgemisch verändert also erfindungsgemäß bevorzugt die Länge und/oder Breite der verformbaren Partikel um die oben genannten bevorzugten Weiten. Durch diese bevorzugte Weitenänderung in Länge und/oder Breite der verformbaren Partikel werden biomechanische Impulse auf die umgebenden Zellen übertragen. Beispielsweise werden Zellen, die an mindestens zwei Haftpunkten an den Partikeln anhaften, durch die Weitenänderung gedehnt. Zellen, die die Partikel umgeben, können beispielsweise aber auch durch die Weitenänderung der Partikel einen Druckimpuls erfahren. Auch ist die Weitergabe der Impulse durch das körpereigene Fibrinnetzwerk möglich. Insbesondere aber werden die Impulse auch über die nicht verformbaren Partikel an die Zellen weitergegeben, da die nicht verformbaren Partikel, die die verformbaren Partikel im Granulatgemisch umgeben, ebenfalls durch die Verformung der verformbaren Partikel bewegt werden.

Die Verformung der verformbaren Partikel bewirkt also nicht nur eine Impulsweitergabe an Osteoblasten, sondern bewegt auch die die verformbaren Partikel umgebenden nicht verformbaren Partikel, so dass die nicht der verformbaren Partikel ebenfalls durch die Bewegung Impulse an die Osteoblasten weitergeben können. Somit können die nicht verformbaren Partikel die Oberfläche zum Anhaften und der Osteoblasten und zum Übertragen der Impulse auf die Osteoblasten, die normaler Weise nur durch die verformbaren Partikel gebildet wird, vergrößern. Dies kann zum Beispiel zu einer Kostenreduktion führen, insbesondere wenn die verformbaren Partikel teurer in der Herstellung sind als die nicht verformbaren Partikel.

Die Impulse können dabei in überraschender und vorteilhafter Weise auch durch die Größe oder die Größenmischungen der verformbaren und/oder der nicht verformbaren Partikel gesteuert werden, beispielsweise in ihrer Intensität, Dauer und/oder Geschwindigkeit gesteuert werden.

Die Impulse können dabei in überraschender und vorteilhafter Weise auch durch das Mischungsverhältnis der verformbaren und der nicht verformbaren Partikel gesteuert werden, beispielsweise in ihrer Intensität, Dauer und/oder Geschwindigkeit gesteuert werden.

Somit kann der Fachmann die Zusammensetzung der Partikel in einem erfindungsgemäßen Granulatgemisch so wählen, dass die Impulsweitergabe auf die Zellen in den von ihm gewünschten Parametern erfolgt, beispielsweise in Bezug auf die Distraktionsdauer, Distraktionsgeschwindigkeit und/oder die Disktraktionsintensität.

Somit kann der Fachmann diese Parameter sehr einfach beeinflussen, nämlich durch eine einfache Änderung der Größe und/oder des Mischungsverhältnisses der Partikel im Granulatgemisch. Darüber hinaus ist ein erfindungsgemäßes Granulatgemisch leicht und kostengünstig herzustellen. Beispielsweise können herkömmliche nicht verformbare Partikel aus dem Stand der Technik ohne großen Aufwand mit den verformbaren Partikeln vermischt werden.

Erfindungsgemäß bevorzugt werden die biomechanischen Impulse mit einer Distraktionsfrequenz von höchstens 1 mm/Tag übertragen. Erfindungsgemäß bevorzugt werden die Dehnungsreize mit einer Distraktionsfrequenz von höchstens 1 mm/Tag übertragen. Erfindungsgemäß bevorzugt werden die Druckreize mit einer Distraktionsfrequenz von höchstens 1 mm/Tag übertragen.

Erfindungsgemäß bevorzugt ist die Degradationskinetik der verformbaren Partikel an das Zeitschema einer mit dem erfindungsgemäßen Granulatgemisch durchzuführenden Distraktion angepasst. Erfindungsgemäß bevorzugt ist das Material der verformbaren Partikel in Abhängigkeit von einer äußeren Krafteinwirkung vordefiniert und kontrolliert dehn-, schrumpf- und/oder verformbar. Das Material kann plastische oder elastische Eigenschaften haben. Diese Eigenschaften des Materials erlauben die erfindungsgemäß vorgesehene Kapazität der verformbaren Partikeln ihr Volumen, reversibel oder irreversibel, vordefiniert und kontrolliert zu ändern.

Erfindungsgemäß bevorzugt ändert sich das Ausgangsvolumen verformbaren Partikel mit einer vorbestimmten Geschwindigkeit. Erfindungsgemäß bevorzugt ist die Geschwindigkeit, mit der sich das Ausgangsvolumen der verformbaren Partikel ändern kann, höchstens so groß, dass die an den Partikeln, also an den verformbaren Partikeln und/oder an den nicht verformbaren Partikeln, haftenden Zellen und/oder die die Partikel umgebenden Zellen höchstens 1,5 mm/Tag, besonders bevorzugt 1,2 mm/Tag, insbesondere 1 mm/Tag, am meisten bevorzugt 0,9 mm/Tag distrahiert und/oder zusammengedrückt werden.

In einer bevorzugten Ausführungsform kann sich das Volumen der verformbaren Partikel vordefiniert und kontrolliert mit einer Geschwindigkeit ändern, bei der eine Dehnung oder Schrumpfung eines Volumens von 1000 µm³ bis 216000 µm³ von höchstens 0,6 mm pro Tag in mindestens eine Raumkoordinate, besonders bevorzugt von höchstens 0,577 mm pro Tag, insbesondere von höchstens 0,55 mm pro Tag, am meisten bevorzugt von höchstens 0,5 mm pro Tag, erfolgt. In einer bevorzugten Ausführungsform kann sich das Volumen vordefiniert und kontrolliert mit einer Geschwindigkeit ändern, bei der eine Dehnung oder Schrumpfung eines Volumens von 1000 µm³ bis 216000 µm³ in mindestens eine Raumkoordinate von mindestens 0,01 mm pro Tag, besonders bevorzugt von mindestens 0,1 mm pro Tag, insbesondere von mindestens 0,2 mm pro Tag, am meisten bevorzugt von mindestens 0,5 mm pro Tag, erfolgt.

In einer bevorzugten Ausführungsform kann sich das Volumen der verformbaren Partikel vordefiniert und kontrolliert mit einer Geschwindigkeit ändern, bei der eine Dehnung oder Schrumpfung eines zwischen 10 µm und 60 µm langen Abschnitts der Raumdiagonalen des Volumens des Quellmittels von höchstens 0,6 mm pro Tag, besonders bevorzugt von höchstens 0,577 mm pro Tag, insbesondere von höchstens 0,55 mm pro Tag, am meisten bevorzugt von höchstens 0,5 mm pro Tag, erfolgt. In einer bevorzugten Ausführungsform kann sich das Volumen vordefiniert und kontrolliert mit einer Geschwindigkeit ändern, bei der eine Dehnung oder Schrumpfung eines zwischen 10 µm und 60 µm langen Abschnitts der Raumdiagonalen des Volumens des Quellmittels von mindestens 0,01 mm pro Tag, besonders bevorzugt von mindestens 0,1 mm pro Tag, insbesondere von mindestens 0,2 mm pro Tag, am meisten bevorzugt von mindestens 0,5 mm pro Tag, erfolgt.

Erfindungsgemäß bevorzugt sind die verformbaren Partikel so gestaltet, dass die Änderung des Ausgangsvolumens der verformbaren Partikel kontinuierlich erfolgen kann. Erfindungsgemäß bevorzugt sind die verformbaren Partikel so gestaltet, dass die Änderung des Ausgangsvolumens der verformbaren Partikel diskontinuierlich erfolgen kann.

Im Zusammenhang mit der vorliegenden Erfindung wird unter "vordefiniert und kontrolliert" eine Änderung des Ausgangsvolumens, insbesondere eine Dehnung oder eine Schrumpfung verstanden, die über eine vorbestimmte Strecke und/oder ein vorbestimmtes Volumen abläuft und deren Geschwindigkeit, also die Dehnungsgeschwindigkeit, Schrumpfungsgeschwindigkeit oder Volumenänderungsgeschwindigkeit ebenfalls vorbestimmt, das heißt bewusst gewählt ist. Erfindungsgemäß kann eine Änderung des Volumens auch lediglich eine Änderung der Form des Volumens sein. Erfindungsgemäß bevorzugt kann auch der Zeitpunkt des Dehnungs-, Schrumpfungs- oder Volumenänderungsbeginns vorbestimmt, das heißt bewusst ausgewählt werden.

Im Zusammenhang mit der vorliegenden Erfindung wird unter einer "Dehnung" eine Vergrößerung der verformbaren Partikel entlang mindestens einer Raumachse, verstanden. Erfindungsgemäß bevorzugt findet die Vergrößerung entlang einer Raumachse statt. Erfindungsgemäß bevorzugt findet die Vergrößerung entlang zweier Raumachsen statt. Erfindungsgemäß bevorzugt findet die Vergrößerung entlang aller drei Raumachsen statt.

Im Zusammenhang mit der vorliegenden Erfindung wird unter einer "Schrumpfung" eine Verkleinerung der verformbaren Partikel entlang mindestens einer Raumachse, bevorzugt entlang einer Raumachse, zweier Raumachsen oder aller drei Raumachsen, verstanden.

Mindestens ein verformbares Partikel ist erfindungsgemäß mit mindestens einem nicht verformbaren Partikel vermischt.

Es kann in einer Ausführungsform insbesondere vorgesehen sein, dass das mindestens eine nicht verformbare Partikel ein Knochenersatzmaterial enthält.

Es kann in einer Ausführungsform insbesondere vorgesehen sein, dass das mindestens eine nicht dehnbare Partikel ein Knochenersatzmaterial enthält.

Es kann in einer Ausführungsform insbesondere vorgesehen sein, dass das Knochenersatzmaterial ein organisches oder ein anorganisches Knochenersatzmaterial ist.

Es kann in einer Ausführungsform insbesondere vorgesehen sein, dass das Knochenersatzmaterial allogener oder autogener Knochen ist.

Es kann in einer Ausführungsform insbesondere vorgesehen sein, dass das mindestens eine nicht verformbare Partikel Hydroxylapatit und/oder Tricalciumphosphat enthält.

Es kann in einer Ausführungsform insbesondere vorgesehen sein, dass das mindestens eine nicht dehnbare Partikel Hydroxylapatit und/oder Tricalciumphosphat enthält.

Es kann in einer Ausführungsform insbesondere vorgesehen sein, dass das mindestens eine nicht verformbare Partikel Hydroxylapatit enthält. Es kann in einer Ausführungsform insbesondere vorgesehen sein, dass das mindestens eine nicht verformbare Partikel Tricalciumphosphat enthält. Es kann in einer Ausführungsform insbesondere vorgesehen sein, dass das mindestens eine nicht verformbare Partikel aus Hydroxylapatit besteht. Es kann in einer Ausführungsform insbesondere auch vorgesehen sein, dass das mindestens eine nicht verformbare Partikel aus Tricalciumphosphat besteht.

Es kann in einer Ausführungsform insbesondere vorgesehen sein, dass das mindestens eine nicht dehnbare Partikel Hydroxylapatit enthält. Es kann in einer Ausführungsform insbesondere vorgesehen sein, dass das mindestens eine nicht dehnbare Partikel Tricalciumphosphat enthält. Es kann in einer Ausführungsform insbesondere vorgesehen sein, dass das mindestens eine nicht dehnbare Partikel aus Hydroxylapatit besteht. Es kann in einer Ausführungsform insbesondere auch vorgesehen sein, dass das mindestens eine nicht dehnbare Partikel aus Tricalciumphosphat besteht.

Es kann in einer Ausführungsform insbesondere vorgesehen sein, dass die Vielzahl der nicht verformbaren, insbesondere nicht dehnbaren Partikel aus dem gleichen oder aus unterschiedlichen Materialien bestehen.

In einer erfindungsgemäßen alternativen Ausführungsform sind die nicht verformbaren Partikel porös. In einer erfindungsgemäßen alternativen Ausführungsform sind die nicht verformbaren Partikel nicht porös.

Erfindungsgemäß bevorzugt ist das mindestens eine nicht verformbare Partikel in vitro hergestellt.

In einer erfindungsgemäßen alternativen Ausführungsform handelt es sich bei den nicht verformbaren Partikeln um am Markt bekannte Partikel wie Bio-Oss® der Firma Geistlich Pharma AG, BONIT matrix® der Firma DOT GmbH oder cydOS® und Ceros® der Firma Mathys AG.

Erfindungsgemäß bevorzugt hat das mindestens eine nicht verformbare Partikel eine Partikelgröße von 0,1 µm bis 50 mm. Erfindungsgemäß bevorzugt hat das mindestens eine nicht dehnbare Partikel eine Partikelgröße von 0,1 µm bis 50 mm.

In einer erfindungsgemäßen Ausführungsform hat das mindestens eine nicht verformbare Partikel eine Partikelgröße von 1 µm bis 50 mm.

In einer erfindungsgemäßen Ausführungsform hat das mindestens eine nicht dehnbare Partikel eine Partikelgröße von 1 µm bis 50 mm.

In einer erfindungsgemäßen Ausführungsform hat das mindestens eine nicht verformbare Partikel eine Partikelgröße von 0,01 mm bis 10 mm.

In einer erfindungsgemäßen Ausführungsform hat das mindestens eine nicht dehnbare Partikel eine Partikelgröße von 0,01 mm bis 10 mm.

In einer erfindungsgemäßen Ausführungsform hat das mindestens eine nicht verformbare Partikel eine Partikelgröße von 0,1 mm bis 10 mm.

In einer erfindungsgemäßen Ausführungsform hat das mindestens eine nicht dehnbare Partikel eine Partikelgröße von 0,1 mm bis 10 mm.

In einer erfindungsgemäßen Ausführungsform beträgt die Partikelgröße 0,2 mm bis 5 mm. In einer erfindungsgemäßen Ausführungsform beträgt die Partikelgröße 0,5 mm bis 5 mm. In einer erfindungsgemäßen Ausführungsform beträgt die Partikelgröße 0,6 mm bis 5 mm. In einer erfindungsgemäßen Ausführungsform beträgt die Partikelgröße 0,5 mm bis 4 mm.

In einer erfindungsgemäßen Ausführungsform beträgt die Partikelgröße mindestens 0,1 mm. In einer erfindungsgemäßen Ausführungsform beträgt die Partikelgröße mindestens 0,2 mm. In einer erfindungsgemäßen Ausführungsform beträgt die Partikelgröße mindestens 0,3 mm. In einer erfindungsgemäßen Ausführungsform beträgt die Partikelgröße mindestens 0,4 mm. In einer erfindungsgemäßen Ausführungsform beträgt die Partikelgröße mindestens 0,5 mm. In einer erfindungsgemäßen Ausführungsform beträgt die Partikelgröße mindestens 0,6 mm.

In einer erfindungsgemäßen Ausführungsform beträgt die Partikelgröße höchstens 10 mm. In einer erfindungsgemäßen Ausführungsform beträgt die Partikelgröße höchstens 5 mm. In einer erfindungsgemäßen Ausführungsform beträgt die Partikelgröße höchstens 4 mm. In einer erfindungsgemäßen Ausführungsform beträgt die Partikelgröße höchstens 2 mm. In einer erfindungsgemäßen Ausführungsform beträgt die Partikelgröße höchstens 1 mm.

Es kann in einer alternativen Ausführungsform insbesondere vorgesehen sein, dass die Partikel der Vielzahl der nicht verformbaren, insbesondere nicht dehnbaren Partikel in einer einzigen Partikelgröße vorliegen.

Es kann in einer alternativen Ausführungsform aber auch vorgesehen sein, dass die Partikel der Vielzahl der nicht verformbaren, insbesondere nicht dehnbaren Partikel mindestens in zwei verschiedenen Partikelgrößen vorliegen. Es kann in einer alternativen Ausführungsform insbesondere auch vorgesehen sein, dass die Partikel der Vielzahl der nicht verformbaren, insbesondere nicht dehnbaren Partikel in zwei verschiedenen Partikelgrößen vorliegen. Es kann in einer alternativen Ausführungsform insbesondere auch vorgesehen sein, dass die Partikel der Vielzahl der nicht verformbaren, insbesondere nicht dehnbaren Partikel in drei verschiedenen Partikelgrößen vorliegen. Es kann in einer alternativen Ausführungsform insbesondere auch vorgesehen sein, dass die Partikel der Vielzahl der nicht verformbaren, insbesondere nicht dehnbaren Partikel in vier verschiedenen Partikelgrößen vorliegen. Es kann in einer alternativen Ausführungsform insbesondere auch vorgesehen sein, dass die Partikel der Vielzahl der nicht verformbaren, insbesondere nicht dehnbaren Partikel in fünf verschiedenen Partikelgrößen vorliegen.

Es kann in einer alternativen Ausführungsform insbesondere auch vorgesehen sein, dass die Partikel der Vielzahl der nicht verformbaren, insbesondere nicht dehnbaren Partikel der Granulatmischung in eins bis zehn, insbesondere in eins bis fünf, aber auch in zwei bis zehn, insbesondere in zwei bis fünf, verschiedenen Partikelgrößen vorliegen.

Es kann in einer alternativen Ausführungsform insbesondere auch vorgesehen sein, dass die Partikel der Vielzahl der nicht verformbaren, insbesondere nicht dehnbaren Partikel der Granulatmischung in einer Vielzahl verschiedener Partikelgrößen vorliegen.

In einer erfindungsgemäßen alternativen Ausführungsform sind die nicht verformbaren Partikel im Granulatgemisch größer als die verformbaren Partikel. In einer erfindungsgemäßen alternativen Ausführungsform sind die nicht verformbaren Partikel im Granulatgemisch bis zu 10-mal größer, insbesondere bis zu 100-mal größer als die verformbaren Partikel.

Erfindungsgemäß bevorzugt ist das mindestens eine nicht verformbare Partikel nicht dehnbar, nicht schrumpfbar und/oder kann seine Form nicht in anderer Weise, beispielweise durch Änderung der Oberflächenkontur, verändern. Erfindungsgemäß bevorzugt ist das mindestens eine nicht verformbare Partikel nicht dehnbar und/oder nicht schrumpfbar. Erfindungsgemäß bevorzugt ist das mindestens eine nicht verformbare Partikel nicht dehnbar und nicht schrumpfbar.

Erfindungsgemäß bevorzugt ist das mindestens eine nicht verformbare Partikel nicht dehnbar.

Erfindungsgemäß bevorzugt handelt es sich bei den nicht verformbaren Partikeln um nicht dehnbare und nicht schrumpfbare Partikel.

Erfindungsgemäß bevorzugt handelt es sich bei den nicht verformbaren Partikeln um starre Partikel. Erfindungsgemäß bevorzugt findet bei den nicht verformbaren Partikeln keine Volumenveränderung statt, beispielsweise bei Kontakt der Partikel mit einer Flüssigkeit.

Erfindungsgemäß bevorzugt ist das Granulatgemisch, insbesondere vor seiner Verwendung, nicht in einer nicht dehnbaren polymeren Matrix, insbesondere in einer Matrix, eingebettet.

In einer erfindungsgemäßen alternativen Ausführungsform enthält das Granulatgemisch zusätzlich zu den verformbaren und nicht verformbaren Partikeln noch Zellen, beispielsweise Stammzellen.

In einer erfindungsgemäßen alternativen Ausführungsform enthält das Granulatgemisch zusätzlich zu den verformbaren und nicht verformbaren Partikeln noch Wachstumsfaktoren. Die Wachstumsfaktoren können entweder an die Partikel, beispielsweise an die verformbaren Partikel und/oder die nicht verformbaren Partikel gebunden sein. Die Wachstumsfaktoren können aber auch nicht an die Partikel gebunden sein.

Die vorliegende Erfindung betrifft auch ein Verfahren zur Herstellung eines erfindungsgemäßen Granulatgemischs, wobei mindestens ein verformbares, insbesondere dehnbares Partikel, insbesondere eine Vielzahl verformbarer, insbesondere dehnbarer Partikel, und mindestens ein nicht verformbares, insbesondere nicht dehnbares Partikel, insbesondere eine Vielzahl nicht verformbaren, insbesondere nicht dehnbarer Partikel, gemischt werden.

Die vorliegende Erfindung betrifft auch ein Verfahren zur Regeneration eines Knochens, wobei mindestens ein erfindungsgemäßes Granulatgemisch in einen Defektbereich eines Knochens eingebracht wird.

Die vorliegende Erfindung betrifft auch medizinische Verfahren wobei ein erfindungsgemäßes Granulatgemisch verwendet wird.

Die Erfindung betrifft also auch die erste medizinische Indikation eines Granulatgemischs aus verformbaren, insbesondere dehnbaren Partikeln und nicht verformbaren, insbesondere nicht dehnbaren Partikeln, insbesondere eines erfindungsgemäßen Granulatgemischs.

In einer erfindungsgemäßen Ausführungsform wird das Granulatgemisch so in einen Defektbereich eines Knochens eingebracht, dass das mindestens eine verformbare, insbesondere dehnbare Partikel mit einer Flüssigkeit in Berührung kommt.

In einer erfindungsgemäßen Ausführungsform wird der Knochendefekt vor dem Einbringen des Granulatgemischs aufgefrischt.

Im Rahmen des erfindungsgemäßen Verfahrens zur Knochenregeneration wird demgemäß in einer bevorzugten Ausführungsform ein Granulatgemisch aus verformbaren und nicht verformbaren Partikeln, insbesondere ein erfindungsgemäßes Granulatgemisch, in einen Defektbereich eines Knochens eingebracht. In diesem Defektbereich wird das Granulatgemisch von einem Blutkoagel eingeschlossen, das heißt die Oberflächen der Partikel kontaktieren die im Blutkoagel enthaltenen autologen Zellen. Nach dem Einbringen des Granulatgemischs in den Defektbereich eines Knochens wird eine Volumenveränderung, also insbesondere eine Volumenverkleinerung oder -vergrößerung, des der verformbaren Partikel durch eine Flüssigkeit ausgelöst. Diese führt zur Ausdehnung und/oder Formveränderung und somit zu der erwünschten biomechanischen Reizung der an die verformbaren und an die nicht verformbaren Partikel angelagerten osteogenen Zellen und so zur Distraktion und damit Knochenregeneration. Erfindungsgemäß bevorzugt erfolgt die Krafteinwirkung innerhalb des Körpers, insbesondere innerhalb des Knochendefekts.

Erfindungsgemäß bevorzugt kann die Volumenänderung der verformbaren Partikel in verschiedenen Größenordnungen liegen. Sie liegt bevorzugt bei etwa 10 % der Längenausdehnung der an die verformbaren Partikel anhaftenden Zellen beziehungsweise Zellgruppen.

Erfindungsgemäß bevorzugt beträgt die Änderung der Dehnungsstrecke mindestens 0,5 µm, besonders bevorzugt mindestens 1 µm, mehr bevorzugt mindestens 10 µm, noch mehr bevorzugt mindestens 100 µm, sehr bevorzugt mindestens 1000 µm, ganz besonders bevorzugt mindestens 10 mm, am meisten bevorzugt mindestens 100 mm.

Erfindungsgemäß bevorzugt beträgt die Änderung der Dehnungsstrecke höchstens 100 mm, besonders bevorzugt höchstens 10 mm, mehr bevorzugt höchstens 1000 µm, noch mehr bevorzugt mindestens 100 µm, sehr bevorzugt höchstens 10 µm, ganz besonders bevorzugt höchstens 1 µm, am meisten bevorzugt höchstens 0,5 µm.

Erfindungsgemäß bevorzugt beträgt die Distraktionsstrecke 5 mm bis 10 mm.

Erfindungsgemäß bevorzugt muss die Distraktionskraft der verformbaren Partikel größer sein als die Schrumpfungskraft des Fibringerüsts oder des Blutkoagels.

Erfindungsgemäß bevorzugt beginnt die Distraktion durch die Verformung, Dehnung oder Schrumpfung der verformbaren Partikel ein Tag nach dem Einbringen des Granulatgemischs in den Knochendefekt. Erfindungsgemäß bevorzugt beginnt die Distraktion durch die Verformung, Dehnung oder Schrumpfung der verformbaren Partikel eine Woche nach dem Einbringen des Granulatgemischs in den Knochendefekt. Der Beginn der Distraktion kann durch eine Umhüllung der verformbaren Partikel bestimmt werden, insbesondere durch die Dicke der Umhüllung.

In einer erfindungsgemäßen Ausführungsform findet die Distraktion über einen Zeitraum von mehreren Tagen oder Wochen statt. In einer erfindungsgemäßen Ausführungsform findet die Distraktion über einen Zeitraum von mehreren Tagen statt. In einer erfindungsgemäßen Ausführungsform findet die Distraktion über einen Zeitraum von mehreren Wochen statt.

In einer erfindungsgemäßen Ausführungsform findet die Distraktion über einen Zeitraum von mindestens einem Tag, insbesondere mindestens 2 Tagen und höchstens 300 Tagen, insbesondere höchstens 100 Tagen statt.

In einer erfindungsgemäßen Ausführungsform findet die Distraktion über einen Zeitraum von mindestens einem Tag statt. In einer erfindungsgemäßen Ausführungsform findet die Distraktion über einen Zeitraum von mindestens 2 Tagen statt. In einer erfindungsgemäßen Ausführungsform findet die Distraktion über einen Zeitraum von mindestens 5 Tagen statt. In einer erfindungsgemäßen Ausführungsform findet die Distraktion über einen Zeitraum von mindestens 10 Tagen statt.

In einer erfindungsgemäßen Ausführungsform findet die Distraktion über einen Zeitraum von höchstens 300 Tagen statt. In einer erfindungsgemäßen Ausführungsform findet die Distraktion über einen Zeitraum von höchstens 100 Tagen statt. In einer erfindungsgemäßen Ausführungsform findet die Distraktion über einen Zeitraum von höchstens 50 Tagen statt.

In einer erfindungsgemäßen Ausführungsform findet die Distraktion über einen Zeitraum von mehreren Tagen, insbesondere über einen Zeitraum von 5 bis 20 Tagen, besonders bevorzugt über einen Zeitraum von etwa 10 Tagen, insbesondere von 10 Tagen, statt.

Erfindungsgemäß bevorzugt beträgt die Geschwindigkeit der Änderung des Volumens mindestens so viel, dass an den Partikeln anhaftende Zellen mindestens 1 µm/Tag distrahiert werden. Erfindungsgemäß bevorzugt beträgt die Geschwindigkeit der Änderung des Volumens so viel, dass an den Partikeln anhaftende Zellen zwischen 0,5 mm/Tag und 1 mm/Tag distrahiert werden. Erfindungsgemäß bevorzugt beträgt die Geschwindigkeit der Änderung des Volumens höchstens so viel, dass an den Partikeln anhaftende Zellen beziehungsweise osteogenes, kallusgebendes Gewebe höchstens 1 mm/Tag distrahiert werden. Eine schnellere Distraktionsfrequenz als 1 mm/Tag führt zur Differenzierung von Bindegewebe anstelle von Knochen. Durch die Volumenveränderung übermitteln die verformbaren Partikel an die im Blutkoagel enthaltenen und an den verformbaren und nicht verformbaren Partikeln adhärierten Zellen biomechanische Reize, die körpereigene Regenerationskräfte auslösen, wodurch sich neues autologes Knochenmaterial bildet. Dieses unterscheidet sich nicht von dem ursprünglichen Knochenmaterial, das den Defekt umgibt. Durch die Volumenveränderung der verformbaren Partikel ergibt sich eine biomechanische Reizübertragung über den ganzen Raum, der von dem Granulatgemisch eingenommen wird, hinweg, sodass auf wesentlich mehr Zellen ein biomechanischer Reiz übertragen wird als bei einer Distraktionsosteogenese aus dem Stand der Technik. Erfindungsgemäß bevorzugt wird der biomechanische Reiz sowohl von den verformbaren Partikeln direkt auf Osteoblasten übertragen als auch von den nicht verformbaren Partikeln auf die Osteoblasten übertragen.

In einer erfindungsgemäßen Ausführungsform findet die Distraktion in alle drei Raumrichtung statt. In einer alternativen erfindungsgemäßen Ausführungsform findet die Distraktion gerichtet in eine oder zwei Raumrichtungen statt. In einer alternativen erfindungsgemäßen Ausführungsform findet die Distraktion gerichtet in eine Raumrichtung statt. Ohne an die Theorie gebunden zu sein, kann es in manchen Situationen vorteilhaft sein, die Distraktion gerichtet in eine Raumrichtung erfolgen zu lassen, damit die Distraktion einer möglichen Ausrichtung der Fasern folgt.

Bei einer erfindungsgemäßen Distraktion können die biomechanischen Reize erfindungsgemäß bevorzugt nicht nur direkt an Osteoblasten, die an den Partikeln anheften, sondern auch indirekt durch Fibroblasten übertragen werden. An die Partikel anhaftende Fibroblasten übertragen erfindungsgemäß bevorzugt den Distraktionsreiz dosiert an Osteoblasten weiter. Ohne an die Theorie gebunden zu sein, werden nach Abschluss der Distraktion auch die Fibroblasten in der so genannten Nullzone zu Osteoblasten und bilden ebenfalls Knochen. Bei einer abnehmenden Distraktionsgeschwindigkeit ändert sich die Anzahl der den Osteoblasten vorgeschalteten Fibroblasten.

Eine Distraktionsosteogenese aus dem Stand der Technik überträgt dagegen biomechanische Reize über eine zweidimensionale Grenzfläche aus Knochen oder einem anderen Material nur an diejenigen Zellen, die diese zweidimensionale Grenzfläche direkt kontaktieren. Ohne an die Theorie gebunden zu sein, kann mit dem erfindungsgemäßen Verfahren sowohl eine Zelldistraktion als auch eine Gewebedistraktion erreicht werden.

Im Zusammenhang mit dieser Erfindung wird unter einer Zelldistraktion die Distraktion einzelner Zellen, insbesondere Osteoblasten, verstanden. Diese einzelnen Zellen lagern sich an die verformbaren oder nicht-verformbaren Partikel an und erfahren durch die Verformung der verformbaren Partikel direkt oder indirekt Distraktionsimpulse. In einer erfindungsgemäßen Ausführungsform beträgt ein Distraktionsimpuls, die eine Zelle, insbesondere Osteoblast, erfährt, von 1 µm bis 10 µm. In einer erfindungsgemäßen Ausführungsform beträgt die Distraktionsstrecke, die eine Zelle, insbesondere Osteoblast, gezogen wird, von 1 µm bis 200 µm. In einer erfindungsgemäßen Ausführungsform beträgt die Distraktionsstrecke, die eine Zelle, insbesondere Osteoblast, gezogen wird, von mindestens 1 µm bis höchstens 10 µm. In einer erfindungsgemäßen Ausführungsform beträgt die Distraktionsstrecke, die eine Zelle, insbesondere Osteoblast, gezogen wird, von mindestens 10 µm bis höchstens 200 µm.

In einer erfindungsgemäßen Ausführungsform beträgt die Geschwindigkeit, mit der eine Zelle, insbesondere ein Osteoblast gezogen wird, mindestens 1 µm/Tag.

Im Zusammenhang mit dieser Erfindung wird unter einer Gewebedistraktion die Distraktion eines Gewebes, beispielsweise eines Knochengewebes, insbesondere eines Kallus, verstanden. Das Gewebe besteht also aus einer Vielzahl von Zellen, insbesondere auch Osteoblasten. Das Gewebe, insbesondere ein Kallus, lagert sich an die verformbaren oder nicht-verformbaren Partikel an und erfährt durch die Verformung der verformbaren Partikel direkt oder indirekt Distraktionsimpulse. In einer erfindungsgemäßen Ausführungsform beträgt ein Distraktionsimpuls, die ein Gewebe, insbesondere ein Kallus, erfährt, von 1 µm bis 1000 µm. In einer erfindungsgemäßen Ausführungsform beträgt die Distraktionsstrecke, die eine Gewebe, insbesondere ein Kallus, gezogen wird, von 10 µm bis 30 cm. In einer erfindungsgemäßen Ausführungsform beträgt die Distraktionsstrecke, die eine Gewebe, insbesondere ein Kallus, gezogen wird, von 10 µm bis 3 cm. In einer erfindungsgemäßen Ausführungsform beträgt die Distraktionsstrecke, die eine Gewebe, insbesondere ein Kallus, gezogen wird, von 10 µm bis 10 mm. In einer erfindungsgemäßen Ausführungsform beträgt die Distraktionsstrecke, die ein Gewebe, insbesondere ein Kallus, gezogen wird, von mindestens 0,2 mm bis höchstens 5 mm.

In einer erfindungsgemäßen Ausführungsform beträgt die Distraktionsstrecke, die eine Gewebe, insbesondere ein Kallus, gezogen wird, mindestens 10 µm. In einer erfindungsgemäßen Ausführungsform beträgt die Distraktionsstrecke, die eine Gewebe, insbesondere ein Kallus, gezogen wird, mindestens 100 µm. In einer erfindungsgemäßen Ausführungsform beträgt die Distraktionsstrecke, die eine Gewebe, insbesondere ein Kallus, gezogen wird, mindestens 1 mm. In einer erfindungsgemäßen Ausführungsform beträgt die Distraktionsstrecke, die eine Gewebe, insbesondere ein Kallus, gezogen wird, höchstens 30 cm. In einer erfindungsgemäßen Ausführungsform beträgt die Distraktionsstrecke, die eine Gewebe, insbesondere ein Kallus, gezogen wird, höchstens 10 cm. In einer erfindungsgemäßen Ausführungsform beträgt die Distraktionsstrecke, die eine Gewebe, insbesondere ein Kallus, gezogen wird, höchstens 3 cm. In einer erfindungsgemäßen Ausführungsform beträgt die Distraktionsstrecke, die eine Gewebe, insbesondere ein Kallus, gezogen wird, höchstens 1 cm. In einer erfindungsgemäßen Ausführungsform beträgt die Distraktionsstrecke, die eine Gewebe, insbesondere ein Kallus, gezogen wird, höchstens 0,5 cm.

In einer erfindungsgemäßen Ausführungsform beträgt die Geschwindigkeit, mit der ein Gewebe, insbesondere ein Kallus gezogen wird, mindestens 10 µm/Tag. In einer erfindungsgemäßen Ausführungsform beträgt die Geschwindigkeit, mit der ein Gewebe, insbesondere ein Kallus gezogen wird, mindestens 0,1 mm/Tag. In einer erfindungsgemäßen Ausführungsform beträgt die Geschwindigkeit, mit der ein Gewebe, insbesondere ein Kallus gezogen wird, mindestens 0,25 mm/Tag. In einer erfindungsgemäßen Ausführungsform beträgt die Geschwindigkeit, mit der ein Gewebe, insbesondere ein Kallus gezogen wird, höchstens 2 mm/Tag. In einer erfindungsgemäßen Ausführungsform beträgt die Geschwindigkeit, mit der ein Gewebe, insbesondere ein Kallus gezogen wird, in etwa 1 mm/Tag.

In einer erfindungsgemäßen Ausführungsform beträgt die Geschwindigkeit, mit der ein Gewebe, insbesondere ein Kallus gezogen wird, mindestens 0,25 mm/Tag und höchstens 2 mm/Tag. In einer erfindungsgemäßen Ausführungsform beträgt die Geschwindigkeit, mit der ein Gewebe, insbesondere ein Kallus gezogen wird, mindestens 0,5 mm/Tag und höchstens 2 mm/Tag. In einer erfindungsgemäßen Ausführungsform beträgt die Geschwindigkeit, mit der ein Gewebe, insbesondere ein Kallus gezogen wird, mindestens 0,5 mm/Tag und höchstens 1,5 mm/Tag.

Die Erfindung stellt somit ein Verfahren bereit, bei dem ein Granulatgemisch aus verformbaren und aus nicht verformbaren Partikeln in einen Knochendefekt eingebracht wird und die verformbaren Partikel im Knochendefekt ihr Volumen und/oder ihre Form verändern. Durch die Volumen- und/oder Formveränderung werden biomechanische Reize auf Zellen, insbesondere Osteoblasten, die sich an der Außenfläche der verformbaren und der nicht verformbaren Partikel befinden, übertragen, wodurch die Zellen zur Knochenbildung angeregt werden. Die Partikel übertragen somit biomechanische Reize zur Ausnutzung körpereigener Regenerationskräfte.

Das erfindungsgemäße Verfahren ist somit eine dreidimensionale Distraktion. Unter einer "dreidimensionalen Distraktion" wird im Zusammenhang mit der vorliegenden Erfindung eine distraktive Knochenregeneration verstanden, bei der biomechanische Reize nicht hur an der Grenzfläche zu einem Knochenfragment, also zweidimensional, übertragen werden, sondern eine Übertragung von Reizen über ein bestimmtes Volumen hinweg, also dreidimensional, stattfindet.

Erfindungsgemäß bevorzugt kann vorgesehen sein, dass die Distraktion entlang einer Raumachse erfolgt. Dies kann zum Beispiel durch Verwendung einer alternativen Ausführungsform der verformbaren Partikel erfolgen, bei dem sich die Länge der Partikel beispielsweise durch einen Faltenbalg ändert.

Das erfindungsgemäße Verfahren nutzt als Bioreaktor die körpereigenen Wundheilungsmechanismen. Die Knochenbildung erfolgt somit unter natürlichen Bedingungen, sodass die notwendigen Aspekte wie Wachstumsfaktoren, Hormone, Zellzusammensetzung implizit berücksichtigt sind. Damit überwindet das erfindungsgemäße Verfahren sowohl Probleme, die durch die hochkomplexe Steuerung bei einer Knochenregeneration entstehen können, als auch die Probleme einer langsamen und komplizierten Knochenregeneration durch Distraktionsverfahren aus dem Stand der Technik.

Erfindungsgemäß bevorzugt wird der Knochendefekt vor dem Einbringen der erfindungsgemäßen Vorrichtung aufgefrischt. Erfindungsgemäß bevorzugt wird bei dem erfindungsgemäßen Verfahren vor dem Einbringen der erfindungsgemäßen Vorrichtung in einen Knochendefekt dieser Defekt chirurgisch aufgefrischt, insbesondere eine Blutung erzeugt. Durch die chirurgische Auffrischung und die erzeugte Blutung bildet sich im Defekt ein Blutkoagel.

Nach der chirurgischen Auffrischung des Knochendefekts wird erfindungsgemäß bevorzugt ein erfindungsgemäßes Granulatgemisch in den Knochendefekt eingebracht. Die Partikel werden von dem gebildeten Blutkoagel umschlossen, insbesondere vollständig umschlossen. Dabei kommen die verformbaren Partikel, beispielsweise auch das die Partikel bildende Quellmittel, erfindungsgemäß bevorzugt in Kontakt mit einer Flüssigkeit, beispielsweise mit einer dem Blut des Blutkoagels.

Erfindungsgemäß bevorzugt wird das Granulatgemisch so in einen Defektbereich eines Knochens eingebracht, dass das Quellmittel der verformbaren Partikel mit einer Flüssigkeit in Berührung kommt.

Erfindungsgemäß bevorzugt verändern die verformbaren Partikel also nach einem definierten Zeitpunkt ihr Volumen. Erfindungsgemäß bevorzugt verändern die verformbaren Partikel nach einem Tag ihr Volumen. Erfindungsgemäß bevorzugt verändern die verformbaren Partikel nach einer Woche sein Volumen. Ohne an die Theorie gebunden zu sein, wird das Blutkoagel nicht schrumpfen, sondern vergrößert sich entsprechend der Volumenvergrößerung der verformbaren Partikel. Die durch das Granulatgemisch aktivierten Zellen können sich zu proliferierenden Osteoblasten umwandeln, die extrazelluläre Matrix produzieren, und es kann ein Kallus entstehen, der anschließend verknöchert. Wenn die verformbaren Partikel erfindungsgemäß bevorzugt bioabbaubar sind, werden diese anschließend resorbiert und/oder metabolisiert. Somit kann sich der Knochendefekt mit Knochengewebe füllen, das erfindungsgemäß bevorzugt durch die beschriebenen biomechanischen Reize des Granulatgemischs entstanden ist. Dabei kann erfindungsgemäß bevorzugt auf Wachstumsfaktoren und andere Substanze neben dem Granulatgemisch verzichtet werden. Das neu gebildete Knochenmaterial unterscheidet sich erfindungsgemäß bevorzugt wenn überhaupt nur gering weder histologisch noch in seinem biologischen beziehungsweise medizinischen Wertigkeit von den ihm umgebenden ursprünglichen Knochen.

Erfindungsgemäß bevorzugt beträgt die Resorptionszeit der verformbaren Partikel etwa 1 bis 2 Jahre, besonders bevorzugt etwa 1,5 Jahre, insbesondere 1,5 Jahre.

Da die verformbaren Partikel erfindungsgemäß bevorzugt bioabbaubar sind, kann der Raum, der durch den Abbau der Vorrichtung entsteht, für die extrazelluläre Matrix genutzt werden. Die Degradation der verformbaren Partikel kann erfindungsgemäß bevorzugt so eingestellt sein, dass bereits nach wenigen Wochen die Partikel, nachdem es die biomechanischen Reize abgegeben hat, degradiert und der entstandene Raum von der extrazellulären Matrix eingenommen wird.

In einer erfindungsgemäßen Alternative können im Rahmen des erfindungsgemäßen Verfahrens verformbare Partikel verwendet werden, deren Ummantelung zelladhäsive Eigenschaften hat. Besonders bevorzugt hat die Oberfläche der Ummantelung zelladhäsive Eigenschaften. Die Oberfläche der Ummantelung spielt für das Anwachsen von Zellen aus dem Blutkoagulat eine Rolle. Eine erfindungsgemäß bevorzugte Adhäsion der Zellen an die Ummantelung kann durch seine Oberflächenchemie und Oberflächenphysik sowie durch die Oberflächentopographie beeinflusst werden. Erfindungsgemäß bevorzugt ist die Oberfläche der Ummantelung hydrophil. Für die einwachsenden Zellen ist die Wechselwirkung zwischen der negativ geladenen Zellmembran und den elektrischen Eigenschaften der Oberfläche der Ummantelung erfindungsgemäß bevorzugt.

Die vorliegende Erfindung betrifft auch die Verwendung eines erfindungsgemäßen Granulatgemischs zur Regeneration eines Knochens, wobei das Granulatgemisch in einen Defektbereich eines Knochens eingebracht wird.

Die vorliegende Erfindung betrifft auch die Verwendung verformbarer, insbesondere dehnbarer Partikel und nicht verformbarer, insbesondere nicht dehnbarer Partikel zur Herstellung eines Granulatgemischs, insbesondere eines erfindungsgemäßen Granulatgemischs, zur Regeneration eines Knochens, wobei das Granulatgemisch in einen Defektbereich eines Knochens eingebracht wird.

Die vorliegende Erfindung betrifft auch ein Granulatgemisch enthaltend mindestens ein verformbares, insbesondere dehnbares Partikel und mindestens ein nicht verformbares, insbesondere nicht dehnbares Partikel, insbesondere ein erfindungsgemäßes Granulatgemisch, zur Verwendung bei der Regeneration eines Knochens.

Die Erfindung betrifft also auch die zweite medizinische Indikation eines Granulatgemischs aus verformbaren, insbesondere dehnbaren Partikeln und nicht verformbaren, insbesondere nicht dehnbaren Partikeln, insbesondere eines erfindungsgemäßen Granulatgemischs, zur Regeneration eines Knochens, insbesondere eines Knochens im Kieferbereich.

Die Erfindung betrifft auch die Verwendung einer erfindungsgemäßen Granulatmischung zur Herstellung eines Kits zur Knochenregeneration.

Die Erfindung betrifft auch ein Kit zur Knochenregeneration, enthaltend eine Vielzahl verformbarer, insbesondere dehnbarer Partikel und eine Vielzahl nicht verformbarer, insbesondere nicht dehnbarer Partikel. Insbesondere betrifft die Erfindung ein Kit zur Knochenregeneration, enthaltend eine erfindungemäße Granulatmischung.

Erfindungsgemäß bevorzugt enthalten die genannten Kits mindestens ein chirurgisches Instrument, besonders bevorzugt mindestens einen Applikator, zum Beispiel eine Spritze, und eine Kapsel zur Aufnahme der Granulatmischung. Erfindungsgemäß bevorzugt enthält der Kit eine Gebrauchsanleitung. Erfindungsgemäß bevorzugt enthält der Kit eine Verpackung, besonders bevorzugt eine Verpackung, die eine sterile Lagerung der Granulatmischung ermöglicht.

Erfindungsgemäß bevorzugt sind die Bestandteile des Kits der erfindungsgemäßen Granulatmischung zugeordnet.

Der erfindungsgemäßen Granulatmischung können also weitere Vorrichtungen wie ein chirurgisches Instrument, eine Gebrauchsanleitung und/oder eine Verpackung zugeordnet sein.

Erfindungsgemäß bevorzugte und alternative Ausführungsformen der erfindungsgemäßen Granulatgemischs sind auch als erfindungsgemäß bevorzugte und alternative Ausführungsformen einer erfindungsgemäßen Verwendungen und als erfindungsgemäß bevorzugte und alternative Ausführungsformen eines erfindungsgemäßen Verfahrens zu verstehen.

Erfindungsgemäß bevorzugte und alternative Ausführungsformen der erfindungsgemäßen Verfahren sind auch als erfindungsgemäß bevorzugte und alternative Ausführungsformen der erfindungsgemäßen Verwendungen und als erfindungsgemäß bevorzugte und alternative Ausführungsformen des erfindungsgemäßen Granulatgemischs zu verstehen.

Weitere vorteilhafte Ausgestaltungen der Erfindung ergeben sich aus den Unteransprüchen. Die Erfindung wird anhand des folgenden Ausführungsbeispiels und der dazugehörigen Figuren näher erläutert.
- Die Figur 1: zeigt schematisch einen Kit umfassend ein Granulatgemisch in einem Applikator in Form einer Spritze.
- Die Figur 2: zeigt schematisch ein in einen Knochendefekt eingebrachtes Granulatgemisch vor und nach der Volumenänderung der in dem Gemisch enthaltenen dehnbaren Partikel.

### Beispiel

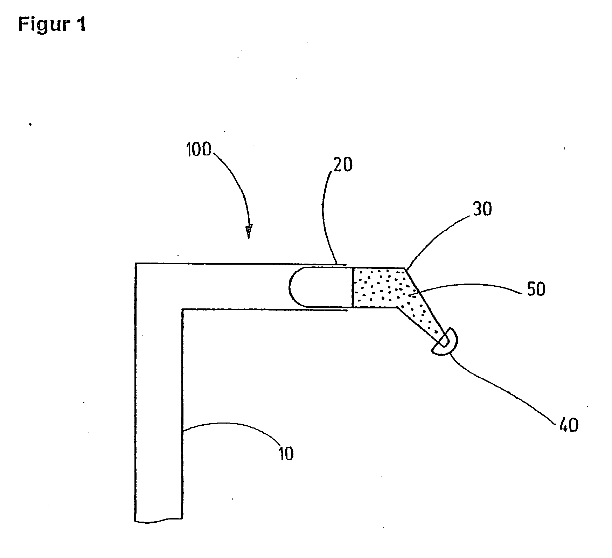

Die Figur 1 zeigt einen Kit 100, enthaltend eine Applikatorspritze 10 aus sterilisierbarem Metall, an deren offenem Ende 20 eine Einmalkapsel 30, zum Beispiel aus Kunststoff, aufgesteckt ist. Die Einmalkapsel 30 ist nach außen hin mit einer Schutzkappe 40 versehen. In der Einmalkapsel 30 befindet sich ein erfindungsgemäßes Granulatgemisch 50 aus verformbaren und nichtverformbaren Partikeln. Das Granulatgemisch wird mittels der Spritze in einen nicht dargestellten Knochendefekt, zum Beispiel im Kieferbereich, eingespritzt.

Der erfindungsgemäße Kit 100 dient dazu, das Granulatgemisch 50 in einen Knochendefekt einzuspritzen. Nach Einbringen in den Knochendefekt ändert sich aufgrund der erfindungsgemäßen Struktur und Zusammensetzung der verformbaren Partikel und durch Flüssigkeitskontakt das Volumen des Quellmaterials der verformbaren Partikel, was zu einer Dehnung, Schrumpfung und/oder Formveränderung der verformbaren Partikel führt. Dadurch werden die nicht verformbaren Partikel und die zwischenzeitlich an die verformbaren und nicht verformbaren Partikel angelagerten Knochenzellen zur Regeneration des Knochens distrahiert.

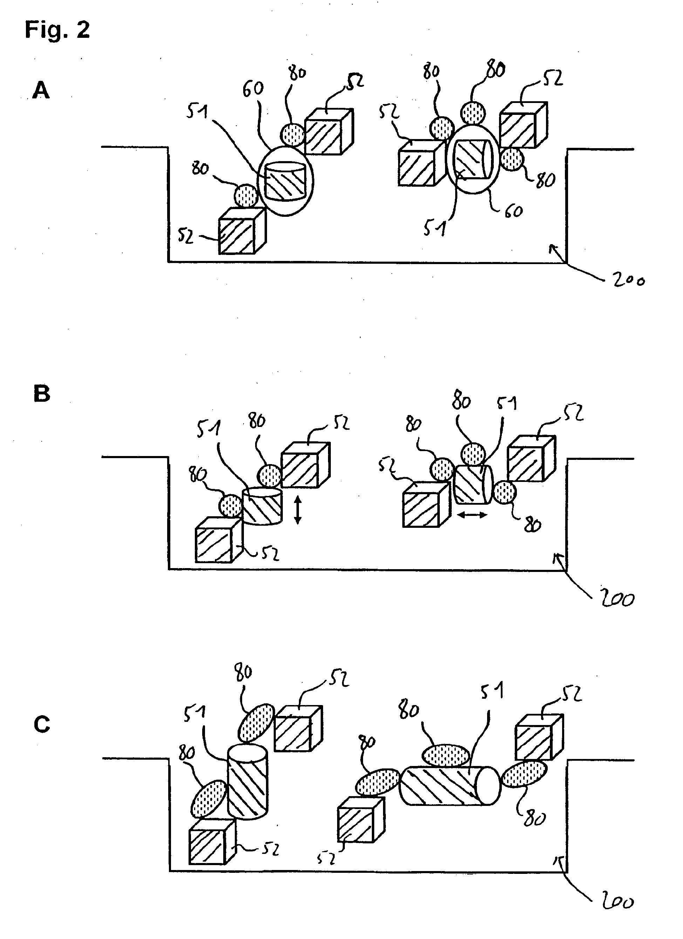

Figur 2A zeigt zwei verformbare Partikel 51 und mehrere nicht verformbare Partikel 52 eines Granulatgemischs 50 in einem Knochendefekt 200, und zwar unmittelbar nach Einbringen des Granulatgemischs 50 in den Knochendefekt 200, zum Beispiel mittels eines Kits 100. Die verformbaren Partikel 51 sind mit Umhüllungen 60 umgeben. Die verformbaren Partikel können beispielsweise aus einem Quellmittel bestehen. Nach Einbringen in den Defekt 200 wird die Umhüllung, die beispielsweise aus Gelatine bestehen kann, biologisch zersetzt und abgebaut. Figur 2B zeigt die Situation nach dem Abbau der Umhüllungen 60. Die verformbaren Partikel 51 kommen nun in direkten Kontakt mit der in dem Knochendefekt befindlichen Flüssigkeit, insbesondere Blut, wodurch sich, wie in Figur 2B durch die Doppelpfeile schematisch dargestellt, das Volumen der verformbaren Partikel 51 in die Längsachse vergrößert. Diese Volumenvergrößerung der verformbaren Partikel 51 führt zu gedehnten Partikeln 51, wie aus Figur 2C erkenntlich. Die Dehnung führt zu einem Wegschieben der umliegenden nicht verformbaren Partikel 52 und somit zu einer Distraktion der an den verformbaren und nicht verformbaren Partikeln 51/52 angelagerten und adhärierten Zellen 80.

## Patentansprüche

1. Granulatgemisch geeignet zur Regeneration eines Knochens, enthaltend mindestens ein dehnbares Partikel und mindestens ein nicht verformbares Partikel, wobei das mindestens eine dehnbare Partikel ein Quellmittel enthält und wobei das Quellmittel des mindestens einen dehnbaren Partikels von einer bioabbaubaren Umhüllung oder von einer bioabbaubaren Ummantelung eingeschlossen ist.

2. Granulatgemisch nach Anspruch 1, wobei das Granulatgemisch eine Vielzahl der dehnbaren Partikel und eine Vielzahl der nicht verformbaren Partikel enthält.

3. Granulatgemisch nach einem der vorhergehenden Ansprüche, wobei das Granulatgemisch aus mindestens einem dehnbaren Partikel und mindestens einem nicht verformbaren Partikel besteht.

4. Granulatgemisch nach einem der vorhergehenden Ansprüche, wobei das Mischungsverhältnis der dehnbaren Partikel und der nicht verformbaren Partikel in dem Granulatgemisch bezogen auf die Partikelanzahl von 1 zu 99 bis 99 zu 1 beträgt.

5. Granulatgemisch nach einem der vorhergehenden Ansprüche, wobei das mindestens eine dehnbare Partikel aus einem Quellmittel besteht.

6. Granulatgemisch nach einem der vorhergehenden Ansprüche, wobei das Quellmittel ein Hydrogel ist.

7. Granulatgemisch nach einem der vorhergehenden Ansprüche, wobei die Dehnung des Quellmittels durch die Aufnahme von Flüssigkeit, bevorzugt von einer Flüssigkeit enthaltend Biomoleküle und/oder Zellen, besonders bevorzugt Blut, durch das Quellmittel ausgelöst wird.

8. Granulatgemisch nach einem der vorhergehenden Ansprüche, wobei das Quellmittel des mindestens einen dehnbaren Partikels aus einem Polysaccharid besteht.

9. Granulatgemisch nach einem der vorhergehenden Ansprüche, wobei das mindestens eine dehnbare Partikel eine Partikelgröße von 0,0001 mm bis 10 mm, insbesondere von 0,1 mm bis 10 mm, hat.

10. Granulatgemisch nach einem der vorhergehenden Ansprüche, wobei die Partikel der Vielzahl der dehnbaren Partikel mindestens in zwei verschiedenen Partikelgrößen vorliegen.

11. Granulatgemisch nach einem der vorhergehenden Ansprüche, wobei das mindestens eine nicht verformbare Partikel ein Knochenersatzmaterial enthält.

12. Granulatgemisch nach einem der vorhergehenden Ansprüche, wobei das mindestens eine nicht verformbare Partikel Hydroxylapatit und/oder Tricalciumphosphat enthält.

13. Granulatgemisch nach einem der vorhergehenden Ansprüche, wobei das mindestens eine nicht verformbare Partikel eine Partikelgröße von 0,01 mm bis 10 mm, insbesondere von 0,1 mm bis 10 mm, hat.

14. Verfahren zur Herstellung eines Granulatgemischs nach einem der Ansprüche 1 bis 13, wobei mindestens ein dehnbares Partikel, insbesondere eine Vielzahl dehnbarer Partikel, und mindestens ein nicht verformbares Partikel, insbesondere eine Vielzahl nicht verformbarer Partikel, gemischt werden.

15. Granulatgemisch nach einem der Ansprüche 1 bis 13, enthaltend mindestens ein dehnbares Partikel und mindestens ein nicht verformbares Partikel zur Verwendung bei der Regeneration eines Knochens.

16. Kit zur Knochenregeneration, enthaltend eine Vielzahl dehnbarer Partikel nach Anspruch 1 und eine Vielzahl nicht dehnbarer Partikel nach Anspruch 1.

## Claims

1. Granulate mixture suitable for regenerating a bone, containing at least one expandable particle and at least one nondeformable particle, wherein the at least one expandable particle contains a swelling agent and wherein the swelling agent of the at least one expandable particle is enclosed by a biodegradable sheating or by a biodegradable casing.

2. Granulate mixture according to Claim 1, wherein the granulate mixture contains a plurality of the expandable particles and a plurality of the nondeformable particles.

3. Granulate mixture according to one of the preceding claims, wherein the granulate mixture is composed of at least one expandable particle and at least one nondeformable particle.

4. Granulate mixture according to one of the preceding claims, wherein the mixing ratio of the expandable particles to the nondeformable particles in the granulate mixture is 1:99 to 99:1, based on the number of particles.

5. Granulate mixture according to one of the preceding claims, wherein the at least one expandable particle is composed of a swelling agent.

6. Granulate mixture according to one of the preceding claims, wherein the swelling agent is a hydrogel.

7. Granulate mixture according to one of the preceding claims, wherein the expansion of the swelling agent as the result of the absorption of liquid, preferably a liquid containing biomolecules and/or cells, particularly preferably blood, is induced by the swelling agent.

8. Granulate mixture according to one of the preceding claims, wherein the swelling agent of the at least one expandable particle is composed of a polysaccharide.

9. Granulate mixture according to one of the preceding claims, wherein the at least one expandable particle has a particle size of 0.0001 mm to 10 mm, in particular 0.1 mm to 10 mm.

10. Granulate mixture according to one of the preceding claims, wherein the particles of the plurality of expandable particles are present in at least in two different particle sizes.

11. Granulate mixture according to one of the preceding claims, wherein the at least one nondeformable particle contains a bone replacement material.

12. Granulate mixture according to one of the preceding claims, wherein the at least one nondeformable particle contains hydroxyapatite and/or tricalcium phosphate.

13. Granulate mixture according to one of the preceding claims, wherein the at least one nondeformable particle has a particle size of 0.01 mm to 10 mm, in particular 0.1 mm to 10 mm.

14. Method for producing a granulate mixture according to one of Claims 1 through 13, wherein at least one expandable particle, in particular a plurality of expandable particles, and at least one nondeformable particle, in particular a plurality of nondeformable particles, are mixed.

15. Granulate mixture according to one of Claims 1 through 13, containing at least one expandable particle and at least one nondeformable particle for use in the regeneration of a bone.

16. Kit for bone regeneration, containing a plurality of expandable particles according to Claim 1 and a plurality of nonexpandable particles according to Claim 1.

## Revendications

1. Mélange de granulés adapté à la régénération d'un os, contenant au moins une particule extensible et au moins une particule indéformable, l'au moins une particule extensible comprenant un agent gonflant et l'agent gonflant de l'au moins une particule extensible étant entouré par une enveloppe biodégradable ou un enrobage biodégradable.

2. Mélange de granulés selon la revendication 1, le mélange de granulés comprenant une pluralité des particules extensibles et une pluralité des particules indéformables.

3. Mélange de granulés selon l'une quelconque des revendications précédentes, le mélange de granulés consistant en au moins une particule extensible et au moins une particule indéformable.

4. Mélange de granulés selon l'une quelconque des revendications précédentes, le rapport de mélange des particules extensibles et des particules indéformables dans le mélange de granulés étant compris entre 1 à 99 et 99 à 1, par rapport au nombre des particules.

5. Mélange de granulés selon l'une quelconque des revendications précédentes, l'au moins une particule extensible consistant en un agent gonflant.

6. Mélange de granulés selon l'une quelconque des revendications précédentes, l'agent gonflant étant un hydrogel.

7. Mélange de granulés selon l'une quelconque des revendications précédentes, l'extension de l'agent gonflant étant lancée par l'absorption d'un liquide, de préférence un liquide contenant des biomolécules et/ou cellules, plus préférablement du sang, par l'agent gonflant.

8. Mélange de granulés selon l'une quelconque des revendications précédentes, l'agent gonflant de l'au moins une particule extensible consistant en un polysaccharide.

9. Mélange de granulés selon l'une quelconque des revendications précédentes, l'au moins une particule extensible présentant une dimension de particules de 0,0001 mm à 10 mm, notamment de 0,1 mm à 10 mm.

10. Mélange de granulés selon l'une quelconque des revendications précédentes, les particules de la pluralité des particules extensibles étant présentes dans au moins deux dimensions de particules différentes.

11. Mélange de granulés selon l'une quelconque des revendications précédentes, l'au moins une particule indéformable comprenant un matériau de substitution osseuse.

12. Mélange de granulés selon l'une quelconque des revendications précédentes, l'au moins une particule indéformable comprenant de l'hydroxyapatite et/ou de la triphosphate de calcium.

13. Mélange de granulés selon l'une quelconque des revendications précédentes, l'au moins une particule indéformable présentant une dimension de particules de 0,01 mm à 10 mm, notamment de 0,1 mm à 10 mm.

14. Procédé pour la fabrication d'un mélange des granulés selon l'une quelconque des revendications 1 à 13, dans lequel au moins une particule extensible, notamment une pluralité des particules extensibles, et au moins une particule indéformable, notamment une pluralité des particules indéformables sont mélangées.

15. Mélange de granulés selon l'une quelconque des revendications 1 à 13, contenant au moins une particule extensible et au moins une particule indéformable, pour l'utilisation dans la régénération d'un os.

16. Kit pour la régénération osseuse, contenant une pluralité des particules extensibles selon la revendication 1 et une pluralité des particules non extensibles selon la revendication 1.
